# EUROPEAN PATENT APPLICATION

(11) **EP 2 100 621 A1**
(43) Date of publication of application: **16.09.2009**
(21) Application number: 08152554.5
(22) Date of filing: 10.03.2008
(51) Int. Cl.: A61K 49/00, A61K 51/00

(54) **Polyether polyol dendron conjugates with effector molecules for biological targeting**

(71) Applicant: mivenion GmbH, 10115 Berlin (DE)
(72) Inventor: Licha, Kai, 14612 Falkensee (AT); Schirner, Michael, 13158 Berlin (DE); Bahner, Malte, 10435 Berlin (DE); Haag, Rainer, 12209 Berlin (DE); Heek, Timm, 13597 Berlin (DE)
(74) Representative: Vossius & Partner

(57) **Abstract**

Subject of the present invention are polyether polyol dendron conjugates comprising a specific polyether polyol dendron moiety, at least one certain fluorescent effector molecule (E). Such polyether polyol dendron conjugates may be used for diagnostic and therapeutic purposes, whereby the optical properties of the at least one certain fluorescent effector molecule are enhanced due to the attachment to the polyether polyol dendron conjugate.

## Description

Subject of the present invention are polyether polyol dendron conjugates comprising a specific polyether polyol dendron moiety, at least one certain fluorescent effector molecule (E). Such polyether polyol dendron conjugates may be used for diagnostic and therapeutic purposes, whereby the optical properties of the at least one certain fluorescent effector molecule are enhanced due to the attachment to the polyether polyol dendron conjugate.

Targeted drug delivery utilizes biological molecules, such as proteins, antibodies and peptides, as carriers for drugs and other molecules with therapeutic effects. The promise of these bioconjugates is to enhance the selectivity of the drug for diseased target tissues, while trying to minimize the side effects of the parent drug. In the past years, a variety of such bioconjugates have entered clinical trials and approval (McCarron PA et al, Molecular Interventions 2005, 5, 368).

The major obstacle in the synthesis of bioconjugates is the physicochemical interaction of the effector molecule with the biomolecule. On the one hand, the target binding properties, stability and solubility of the biomolecule have to be maintained after chemical modification, while on the other hand, a high number of effector molecules to be transported have to be coupled to achieve a most effective payload delivery. The nature of the linker chemistry used for conjugation of drugs to carrier molecules has an important impact on the resulting properties. The properties of the linker determine, for example, the position of the drug on the carrier molecule, the number per carrier molecule, and the interaction with the carrier molecule based on the degree of hydrophilicity. Second, the linker can be stable or it can be made cleavable either by lower pH or by enzymes (esterases, proteases). In "Bioconjugate Techniques" (G. Hermanson, Academic Press, 1996) linker chemistry is described comprehensively.

For fluorescent dyes, it has been well described that coupling to peptidic structures leads to fluorescence quenching (Bouteiller C, Bioconj. Chem 2007, 18, 1303-1317, Tung CH, Biopolymers 2004, 76, 391-403).

After coupling to antibodies and proteins, photosensitizers undergo loss of absorbance (e.g. WO 2007/042775, Fig. 28) and exhibit decreased efficacy to produce singlet oxygen after excitation (e.g. Stefflova K. et al., Curr Med Chem 2007, 14, 2110-2125). Coupling of higher numbers of photosensitizer molecules to antibodies caused insolubility (e.g. above 4 molecules m-THPC to Mab, Cane. Res. 1999, 59, 1505-1513) and coupling occurred non-directed and statistically at various positions in the sequence of the antibody. Such direct coupling of effector molecules to undefined positions in a biological targeting molecule, such as an antibody, leads to a decreased affinity of the targeting molecule to its target and often results in insufficient solubility of the labeled protein. Insufficient insolubility requires the addition of organic solvents, such as DMSO, to re-dissolve the labeled protein. This is however disadvantageous due to the toxicity of organic solvents. It is known that fluorescent dyes exhibit strong molecular interactions, also in the presence of protein structures and thus have a high tendency to aggregate or form complex structures. As a result, the optical properties of such fluorescent dyes are impaired, in particular the fluorescence quantum yield and the singlet oxygen yield.

Polymeric hyperbranched polyglycerols have been described in broad versatility as components for drug solubilization, transport and delivery, as well as in material science, e.g. in Haag R et al. (J. Am. Chem. Soc. 2000; 122:2954-2955, Haag R et al. (Angew Chem Int Ed Engl 2006 45:1198-215), Frey H (J Biotechnol 2002, 90:257-67). Such hyperbranched polyglycerols are generated by a polymerization procedure and are thus not of single, defined molecular weight.

WO 2005/023844 describes defined polyglycerol dendrimers as modifiers for amphiphilic molecules.

Other examples of defined polyethers include Yang M (JACS 2005, 127, 15107), Fulton DA (Chem Commun 2005, 474), and Grayson SM (JACS 2000, 122, 10339), who used a C₄-unit.

Kolhe P et al. (Pharm. Res. 2004, 21, 2185) published polyethers (C₄ units) labeled with the drug ibuprofen and the fluorescent dye fluorescein-isothiocyanate. The polyglycerol described therein is structurally not defined. Effects on the optical properties of the dye were not disclosed.

The combination of polymers with imaging agents was reported repeatedly including mainly agents for magnetic resonance imaging, e.g, Gd-complexes [Caruthers SD et al., Methods Mol Med. 2006, 124:387-400]. Respective systems comprising optical effector molecules are not known.

The objective of the present invention was therefore to provide an improved conjugate platform for optical effector molecules. It was surprisingly found that a novel class of dendritic molecules based on polyether polyols is suited particularly as conjugate platform for optical effector molecules from the class of fluorescent dyes (diagnostic effectors) and photosensitizers (therapeutic effectors). These polyether polyol dendrons may be monodisperse, or even perfect synthetic dendrons. Such conjugates exhibit minimized aggregation, enhanced hydrophilicity and improved solubility in aqueous media, as well as the ability to conjugate more than one effector molecule at a time to biological targeting molecules via the dendritic structure of the polyether polyol. Depending on the structure of the biomolecule, the polyether polyol conjugates are suited to be coupled to a defined position in the biomolecule in a single synthesis step. This results in a conjugate with a biological targeting molecule carrying multiple effector molecules, which are not placed statistically over the entire biomolecule structure hampering the activity of the biomolecule, but at a predefined position.

The polyether polyols were found to be able to optimize the interaction of the effector with its environment in solution in the form of effector-polyether polyol conjugates. According to the present invention it was surprisingly found that the polyether polyol dendrons lead to advantageous properties of diagnostic effector molecules (enhanced fluorescence quantum yields), as well as therapeutic effector molecules (enhanced singlet oxygen yield) together with increased water solubility, the prevention of precipitation from the aqueous solution.

It was furthermore found that the covalent attachment of conjugates of polyether polyol dendrons and fluorescent dyes to biological targeting molecules, in particular peptides and antibodies, leads to advantageous properties of the biological targeting molecules. After labeling of an antibody with a cyanine dye-polyether polyol conjugate, the resulting stability and solubility of the labeled protein was increased compared to the same antibody labeled with the corresponding dye alone. Similar to the effector-polyether polyol conjugates, the polyether polyol dendron conjugates with biological targeting molecules lead to advantageous properties of diagnostic effector molecules (enhanced fluorescence quantum yields) as well as therapeutic effector molecules (enhanced singlet oxygen yield) together with increased water solubility and the prevention of organic solvents, when conjugated to antibodies.

Further subject is the additional conjugation with a radiometal chelate as radiodiagnostic or radiotherapeutic effectors.

Subject of the present invention is a polyether polyol dendron conjugate, comprising
a) a polyether polyol dendron moiety (D),
b) at least one effector molecule (E) selected from the group of fluorescent dyes or photosensitizers, or a radioactive effector molecule, selected from the group of radiometal complexes or radiometal chelates,
wherein the polyether polyol dendron moiety (D) consists of a core unit comprising a C₃-C₅ hydrocarbon backbone, at least one functional group R¹, and furthermore having two hydroxyl groups to each of which n = 1-10 generations of dendritically repeating building block units may be covalently linked, whereby the building block units furthermore comprise a C₃-C₅ hydrocarbon backbone and two hydroxyl groups,
wherein the hydroxyl groups of the outermost generation of building block units (exterior units or shell), are replaced by one or more independently selected functional groups R²,
wherein the building block units may all be of the same type or may be selected from more than one type of different units,
wherein the exterior units may all be of the same type or may be selected from more than one type of different units, and
wherein the dendron conjugate exhibits a fluorescence quantum yield in aqueous media of at least 5%, preferably at least 10%, more preferably at least 15%, most preferably at least 20% in case (E) is a fluorescent dye, and a singlet oxygen yield in aqueous media of at least 30%, preferably at least 50%, more preferably at least 70% in case (E) is a photosensitizer.

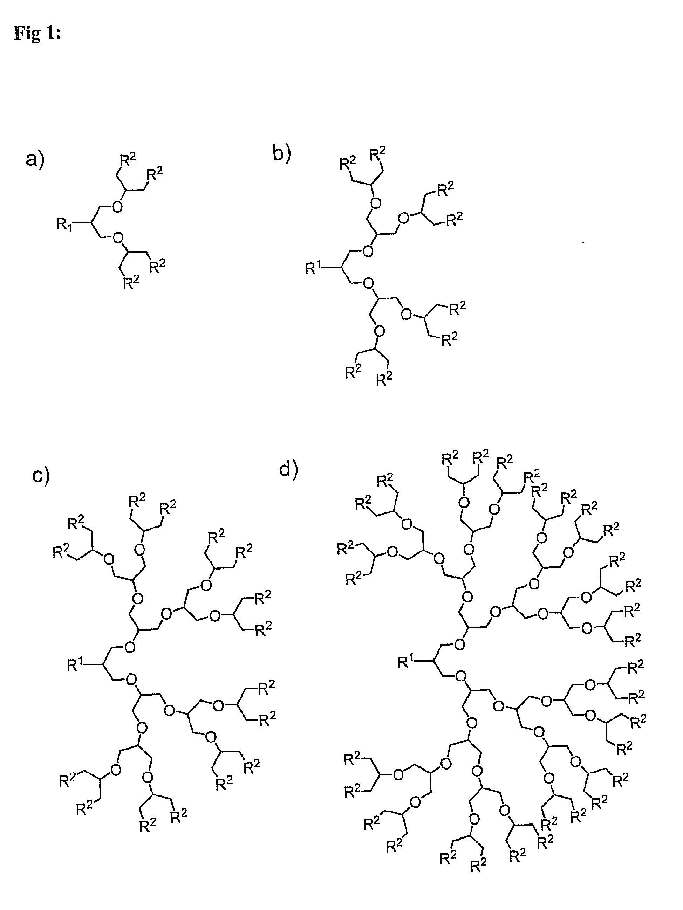

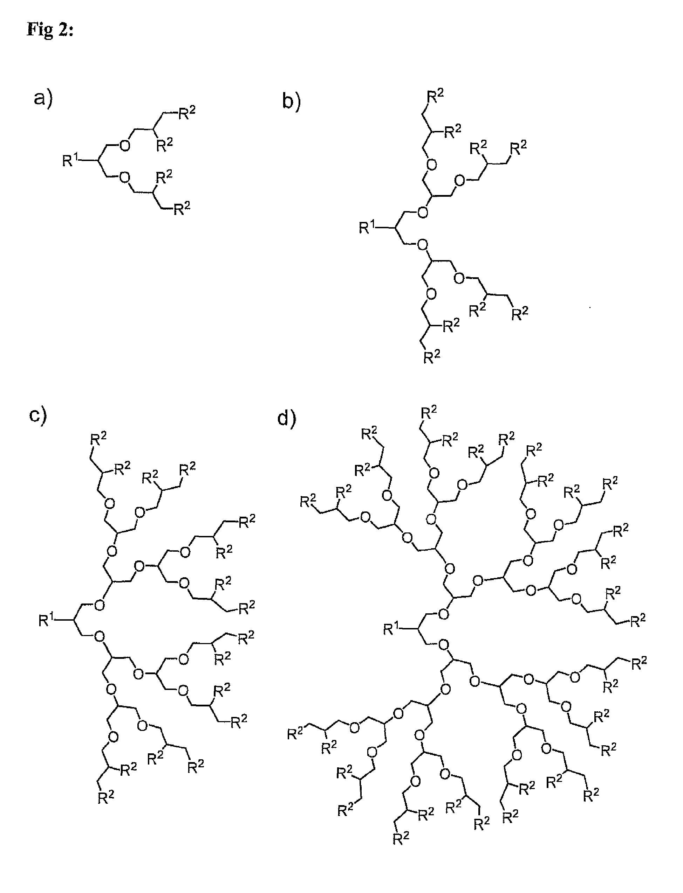

Exemplary representations of dendron moieties according to the present invention are pictured in Figures 1 and 2. However, the dendrons according to the present invention are by no means limited to the specific embodiments shown in these figures.

In a preferred embodiment the polyether polyol dendron moiety (D) is fully synthetic.

Fully synthetic means that the dendron structure is build up synthetically from smaller units (which may be of synthetic, semi-synthetic or natural origin), so that the exact structure of the dendron may be tailored in the desired form.

Preferred is a C₃ hydrocarbon backbone.

In a more preferred embodiment the polyether polyol dendron moiety (D) is a monodisperse polyether polyol dendron.
The term "monodisperse" is used in the meaning generally understood in the field of dendrimer chemistry. This means that a given dendrimer or dendron has a narrow molecular weight distribution in which one particular species of a defined molecular weight is predominantly present. More specifically, the one particular species is present in a ratio of 90% or more, based on the total amount of dendrimer or dendron, preferably 95% or more.

In another more preferred embodiment the polyether polyol dendron moiety (D) is a perfect polyether polyol dendron.

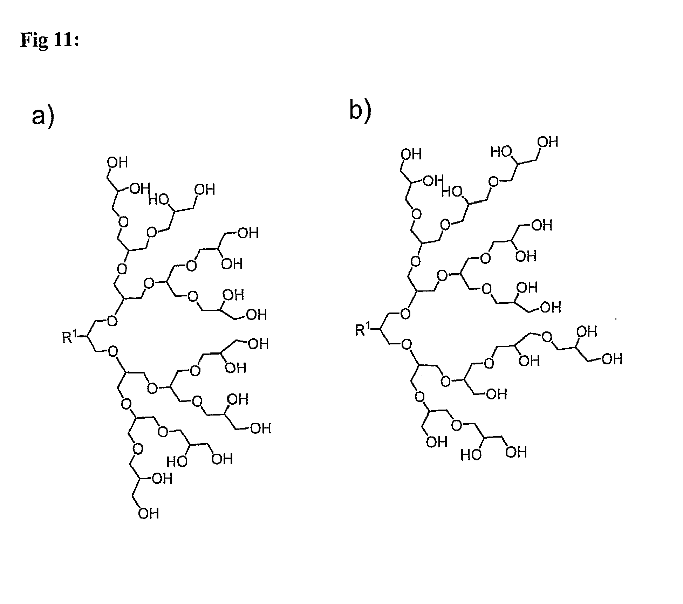

The term "perfect dendron" describes a monodisperse, highly symmetric dendron in which all dendron generations contain the maximum number of theoretically possible building block units. Figure 11a is a schematic representation of such perfect dendrons. In comparison, Figure 11b is a schematic representation of a non-perfect dendron.

In another more preferred embodiment the polyether polyol dendron moiety (D) has a molecular weight of 70 to 6000 g/mol, even more preferred 200 to 4000, yet even more preferred 300 to 3000 g/mol.

In another more preferred embodiment the integer n relating to the polyether polyol dendron moiety (D) has a value of 2-8, more preferred 2-5, most preferred 3 or 4.

In another more preferred embodiment the at least one functional groups R¹ are independently selected from the group comprising -OH, -OSO₃H, -OSO₃Na, -NH₂, -N₃, -COOH, -SH, -SO₃⁻, -C≡C, -C₁₋₂₀-alkyl, -CONH-C₁₋₂₀-alkyl, -NHC(O)-C₁₋₂₀-alkyl and -O-C₁₋₂₀-alkyl,
wherein the C₁₋₂₀-alkyl group is a branched or linear alkyl group in which one or more (preferably one to three) non-consecutive methylene groups may be replaced by a group selected from the group comprising O, S, NH, C(O)NH, SO₂, SO, aryl, ethene or ethyne, and wherein said alkyl is substituted with at least one (preferably 1 to 3) groups selected from the group comprising -OH, -OSO₃H, -OSO₃Na, NH₂, -N₃, -COOH, -SH, -SO₃⁻, -C≡C,.

In another more preferred embodiment the functional groups R² are independently selected from the group comprising -OH, -OSO₃H, -OSO₃Na, NH₂, -N₃, -COOH, -SH, -SO₃⁻, -C≡C, -C₁₋₂₀-alkyl, -CONH-C₁₋₂₀-alkyl, -NHC(O)-C₁₋₂₀-alkyl and -O-C₁₋₂₀-alkyl, wherein the C₁₋₂₀-alkyl group is a branched or linear alkyl group in which one or more (preferably one to three) non-consecutive methylene groups may be replaced by a group selected from the group comprising O, S, NH, C(O)NH, SO₂, SO, aryl, ethene or ethyne, and wherein said alkyl is substituted with at least one (preferably 1 to 3) groups selected from the group comprising -OH, -OSO₃H, -OSO₃Na, NH₂, -N₃, -COOH, -SH, -SO₃⁻, -C≡C,.

From the above-mentioned groups R¹ and R², the even more preferred combinations of groups are: R¹ = -OH, R² selected as described above; as well as R¹ selected from the group comprising -OH, NH₂, -C₁₋₁₂alkyl-NH₂, -O-C₁₋₁₂alkyl-NH₂, -COOH, -C₁₋₁₂alkyl-COOH, -O-C₁₋₁₂alkyl-COOH, -SH, -C₁₋₁₂alkyl-SH, -Q-C₁₋₁₂alkyl-SH, R² = OH.

It is yet even more preferred that R¹ = -O-C₁₋₆-alkyl, substituted with one -NH² or -COOH, R²=-OH.
The groups R¹ and R² mentioned in the context of the present invention may be covalently coupled to a reactive group of a further molecule (such as (D), (E), (L) or (B)), whereby a group selected from disulfide, amide, amine, ether, thioether, thioester, carboxyester, sulfonylester, sulfonamide, urea, carbamate, thiocarbamate, triazol results.

In another embodiment, the core unit has more than one functional group R¹ and said groups R¹ are different from one another.

In another embodiment the core unit has one functional group R¹.

In another more preferred embodiment the core unit, the building block units and the exterior units are structurally derived from glycerol, whereby in the core unit one of the OH groups of the glycerol is replaced by a group R¹ as described above, and whereby in the exterior units two of the OH groups are replaced by independently selected groups R² as described above,. The resulting dendron may hereby be seen as a polyglycerol.

It is apparent to a person skilled in the art, that the polyether polyol dendrons according to the present invention, which are composed of polyether units (which are glycerol units in a more preferred embodiment), comprise a multitude of ether bonds, originating from the reaction of the hydroxyl groups of the polyether polyol units (compare example 1 for a schematic representation).

In another embodiment the building block units within each of the dendron generations in the polyether polyol dendron (D) are all of the same type, and may be of a different type in different dendron generations.

In another more preferred embodiment the building block units in the polyether polyol dendron moiety (D) are all of the same type.

In another preferred embodiment, when the exterior units in the polyether polyol dendron moiety have more than one functional group R², said groups R² are different from one another.

In another more preferred embodiment the exterior units in the polyether polyol dendron moiety have two functional groups R².

In another more preferred embodiment the exterior units in the polyether polyol dendron moiety (D) are all of the same type.

In yet another preferred embodiment at least one of the effector molecules (E) is an optical effector molecule with diagnostic function, comprising a fluorescent dye with a fluorescence emission in the UV/visible (400-800 nm) or near-infrared (700-1000 nm) spectral range.

More preferably, the optical effector molecule with diagnostic function is selected from the group comprising NBD, fluoresceins, rhodamines, perylene dyes, croconium dyes, squarylium dyes, polymethine dyes, indocarbocyanine dyes, indodicarbocyanine dyes, indotricarbocyanine dyes, merocyanine dyes, phthalocyanines, naphthalocyanines, triphenylmethine dyes, croconium dyes, squarylium dyes, benzophenoxazine dyes, benzophenothiazine dyes, and derivatives thereof.

Even more preferably, the optical effector molecule with diagnostic function is selected from the group comprising polymethine dyes, indocarbocyanine dyes, indodicarbocyanine dyes, indotricarbocyanine dyes, merocyanine dyes, phthalocyanines, naphthalocyanines, triphenylmethine dyes, croconium dyes, squarylium dyes, and derivatives thereof.

Yet even more preferably, the optical effector molecule with diagnostic function is selected from the group comprising indocarbocyanine, indodicarbocyanine, indotricarbocyanine dyes and derivatives thereof.

Most preferably, the optical effector molecule with diagnostic function is a fluorescent dye comprising the structural elements of indocyanine green (ICG) and derivatives thereof.

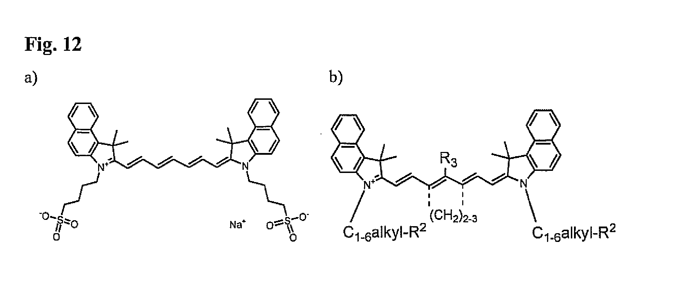

Hereby, the derivatives of ICG are preferably structurally described by
a) replacement of one or two sulfobutyl chains at the indol nitrogen by -C₁₋₆-alkyl-R², whereby R² is as described above; and/or
b) replacement of the polymethine chain by a substituted polymethine chain with a residue R³ at the central carbon atom, whereby the two adjacent carbons atoms may form a 5- or 6-membered ring together with the three carbon atoms of the polymethine chain, whereby R³ is selected from the group comprising -C₁₋₆-alkyl-R², -phenyl-C₁₋₆alkyl-R², -S-phenyl- C₁₋₆alkyl-R², -O-phenyl-C₁₋₆alkyl-R², whereby R² is as described above, and/or
c) substitution of the exterior benzindol rings with one or more groups independently selected from -SO₃⁻Na⁺, -COOH or -OH. The structure of such derivatives is exemplified in Figure 12, which shows the structure of ICG and of derivatives in accordance with the present invention.

It is more preferred that the polymethine chain has a residue R³ as described above at the central carbon atom, wherein R² is -COOH or -SO₃⁻Na⁺, and wherein the two adjacent carbons atoms may form a 5- or 6-membered ring together with the three carbon atoms of the polymethine chain.

Examples of synthesis routes leading to optical effector molecule with diagnostic function which may be used in accordance with the present invention are published in "Topics in Applied Chemistry: Infrared absorbing dyes" Ed. M. Matsuoka, Plenum, N.Y. 1990, "Topics in Applied Chemistry: The Chemistry and Application of Dyes", Waring et al., Plenum, N.Y., 1990, J. Org. Chem. 60: 2391-2395 (1995), Lipowska et al. Heterocyclic Comm. 1: 427-430 (1995), Fabian et al. Chem. Rev. 92: 1197 (1992), WO 96/23525, Strekowska et al. J. Org. Chem. 57: 4578-4580 (1992), Bioconjugate Chem. 16:1275-128 (2005).

In yet another preferred embodiment at least one of the effector molecules (E) is an optical effector molecule with therapeutic function, comprising a photosensitizer with phototherapeutic efficacy after excitation in the UV/visible (400-800 nm) or nsar-infrared (700-1000 nm) spectral range.

More preferably, the photosensitizer is selected from the group comprising tetrapyrroles, porphyrins, sapphyrins, chlorins, tetraphenylporphyrins, tetraphenylchlorins, bacteriochlorins, tetraphenylbacteriochlorins, pheophorbides, bacteriopheophorbides, pyropheophorbides, bacteriopyropheophorbides, purpurinimides, bacteriopurpurinimides, benzoporphyrins, phthalocyanines, naphthalocyanines and derivatives thereof.

Even more preferably, the photosensitizer is selected from the group comprising pheophorbide a, pyropheophorbide a, 3-acetylpheophorbide a, 3-acetylpyropheophorbide a, purpurin-18-*N*-alkylimide, purpurin-18-*N-*hydroxylimide, 3-acetylpurpurin-18-*N-*alkylimide, 3-acetylpurpurin-18-*N*-hydroxylimide, chlorine e6, Sn-chlorine e6, m-tetrahydroxyphenylchlorin (m-THLC) and benzoporphyrin derivative, benzoporphyrin derivative monoacid (BPD-MA, verteporfin).

Yet even more preferably, the photosensitizer is selected from the group comprising pheophorbide a, pyropheophorbide a, 3-acetylpheophorbide a, 3-acetylpyropheophorbide a, purpurin-18-*N*-alkylimide, purpurin-18-*N*-hydroxylimide, 3-acetylpurpurin-18-*N*-alkylimide, 3-acetylpurpurin-18-*N*-hydroxylimide and chlorine e6, benzoporphyrin derivative, benzoporphyrin derivative monoacid (BPD-MA, verteporfin).

Most preferably, the photosensitizer is selected from the group comprising pheophorbide a, pyropheophorbide a, purpurin-18-*N-*alkylimide, purpurin-18-*N-*hydroxylimide and chlorine e6, verteporfin.

In another preferred embodiment, the photosensitizer has two structural elements, which allow the modification with two polyether polyol dendrons. For instance, pheophorbide a, pyropheophorbide a and purpurinimides have a vinyl group at position 3 in addition to their carboxylic acid group, which can be both independently modified and with a further molecule (such as (D), (E), (L) or (B)).

Examples of synthesis routes leading to photosensitizers which may be used in accordance with the present invention are published in WO 2003/028628, US 2005/0020559, Zheng G et al, J Med Chem 2001, 44, 1540-1559; Li G et al., J. Med. Chem. 2003, 46, 5349-5359; Lunardi CN et al., Curr Org Chem 2005, 9, 813-821; Chen Y et al., Curr Org Chem 2004, 8, 1105-1134.

In yet another preferred embodiment optionally at least one of the effector molecules (E) is a radiolabeled complex comprising a radionuclide and a chelating structure selected from tetraazacyclododecane chelates and makrocyclic or open-chain aminocarboxylic acids.

More preferably, the radiolabeled complex comprises a chelating agent selected from the group comprising 1,4,7,10-tetraazacyclododecane-N,N',N",N"'-tetraacetic acid (DOTA), 1,4,7,10-tetraazacyclododecane-N,N',N"-triacetic acid (DO3A),
1-oxa-4,7,10-triazacyclododecane-N,N',N"-triacetic acid (OTTA),
trans(1,2)-cyclohexanodiethylentriamine- pentaacetic acid (CDTPA),
N,N,N',N",N"-diethylentriamine-pentaacetic acid (DTPA), ethylenediamine-tetraacetic acid (EDTA), N-(2-hydroxy)ethylen-diamine triacetic acid, nitrilotriacetic acid (NTA), N,N-di(2-hydroxyethyl)glycine and derivatives thereof and
a radionuclide selected from ⁹⁰Y, ^{99m}Tc, ¹¹¹In, ⁴⁷Sc, ⁶⁷Ga, ⁵¹Cr, ^{177m}Sn, ⁶⁷Cu, ¹⁶⁷Tm, ⁹⁷Ru, ¹⁸⁸Re, ¹⁷⁷Lu, ¹⁹⁹Au, ²⁰³Pb, ¹⁴¹Ce, ⁸⁶Y, ^{94m}Tc, ^{110m}In, ⁶⁸Ga, ⁶⁴Cu.

Even more preferably, the radiolabeled complex is selected from the group comprising 1,4,7,10-tetraazacyclododecane-N,N',N",N"'-tetraacetic acid (DOTA), 1,4,7,10 tetraazacyclododecane-N,N',N"-triacetic acid (DO3A), N,N,N',N",N"-diethylentriamine-pentaacetic acid (DTPA) and a radionuclide selected from ⁹⁰Y, ^{99m}Tc, ¹¹¹In, ⁶⁸Ga, ⁸⁶Y, ⁶⁴Cu,

Most preferably, the radiolabeled complex is selected from the group comprising 1,4,7,10-tetraazacyclododecane-N,N',N",N"'-tetraacetic acid (DOTA) and a radionuclide selected from ¹¹¹In, ⁶⁸Ga, ⁸⁶Y, ⁶⁴Cu

In another particularly preferred embodiment the polyether polyol dendron conjugate furthermore comprises one or more biological targeting molecules (B).

Even more particularly preferred, the one or more biological targeting molecules (B) are independently selected from the group comprising a peptide, a peptidomimetic molecule, an antibody or a fragment thereof, an antibody mimetic (e.g. Fv, Fab, Fab', F(ab')₂, Fabc, Facb; single chain antibodies, e.g. single chain Fvs (scFvs); and diabodies), a protein, an oligonucleotide, a peptide-oligonucleotide, a carbohydrate.

Most preferred, the one or more biological targeting molecules (B) are independently selected from the group comprising an antibody, an antibody mimetic, a peptide and a peptidomimetic molecule.

Suited antibodies, antibody fragments, and antibody mimetics are for example those with a binding component against angiogenesis targets. These biological targeting molecules are ligands against receptors expressed in the newly formed vasculature and vascularizing structures. Examples are antibodies against ED-B-fibronectin (described in Cancer Res. 1999, 59:347-352; EP 0 344 134 B1, WO 97/45544 A1, WO 99/5857 A2, WO 01/62800 A1). Particularly preferred are L19, E8, AP 38 and AP 39. Another type of antibodies are ligands to VEGF receptor (VEGFR-1, VEGFR-2 and VEGF-R3), e. g. Bevacizumab (Avastin™, rhumAb-VEGF), ranibizumab (Lucentis™), mAb 6.12, IMC-2C6, IMC-1121, HF4-3C5, KM-2550 and Salgaller ML (2003) Current Opinion in Molecular Therapeutics 5(6):657-667. Other antibodies bind to endoglin (CD-105), e. g. SN6h, SN6, SN6a, SN6j, P3D1, P4A4, 44G4, GRE, E-9, CLE-4, RMAC8, PN-E2, MAEND3, TEC4, TEC11, A11, 8E11.

Suited peptides and peptidomimetics are those binding to G-protein coupled receptors, e. g. the somatostatin receptor, bombesin receptor, neurotensin receptor, VIP receptor, neuropeptide Y receptor; as well as those binding to integrins (RGD-peptides and peptide mimetics), the uPA-receptor (upAR), the VEGF receptor, EGF.

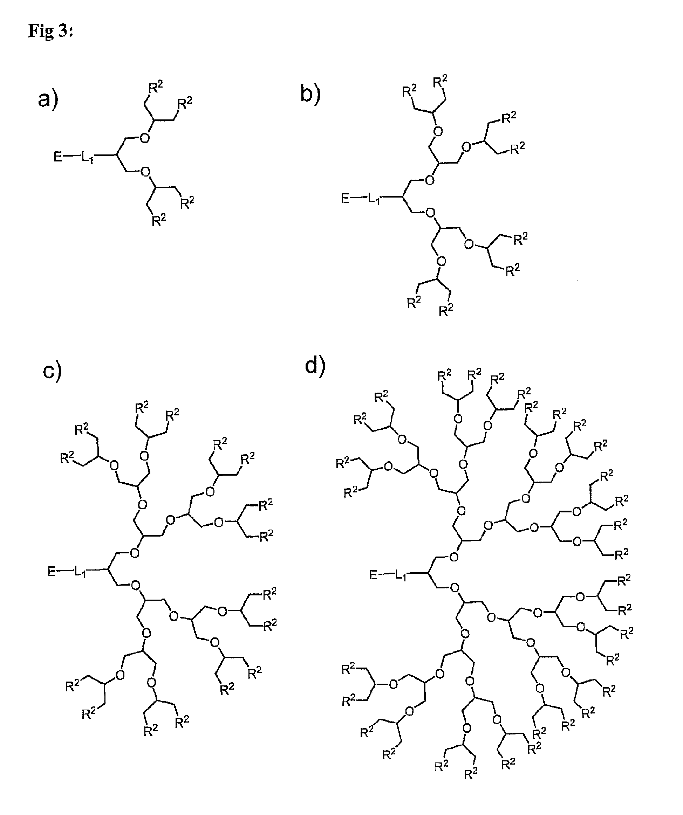

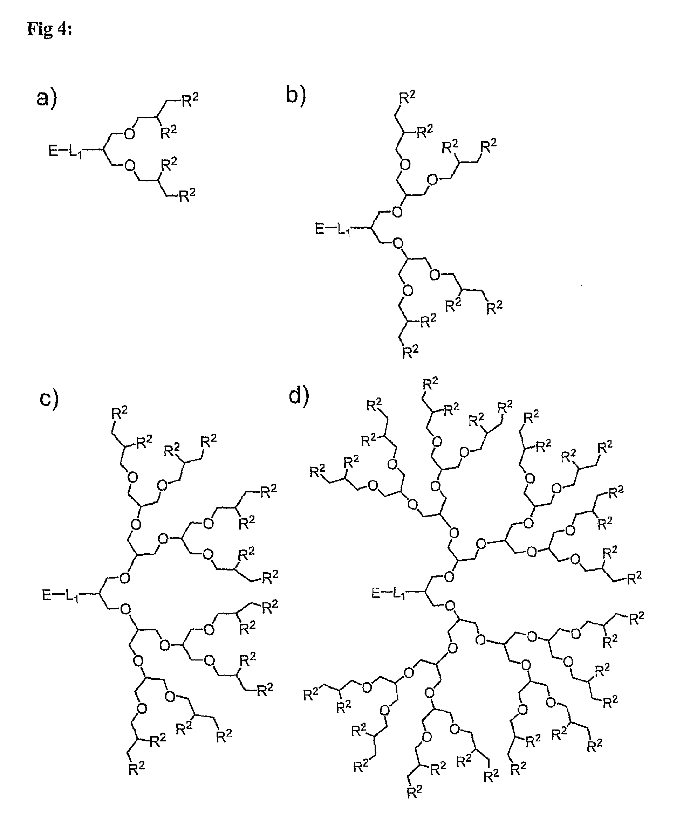

In a preferred embodiment the at least one effector molecule (E) is covalently linked to a functional group R¹ of the core unit and/or to one or more of the functional groups R² of one or more of the shell units, and
wherein one or more biological targeting molecules (B) may be covalently linked to a functional group R¹ of the core unit and/or to one or more shell units.
Exemplary representations of such dendron conjugates in which (E) is covalently linked to the functional group R¹ of the core unit are pictured in Figures 3 and 4.

In another preferred embodiment the at least one effector molecule (E) is covalently linked to a functional group R¹ of the core unit and/or to one or more of the functional groups R² of one or more of the shell units, and
wherein one biological targeting molecule (B) may be covalently linked to a functional group R¹ of the core unit.

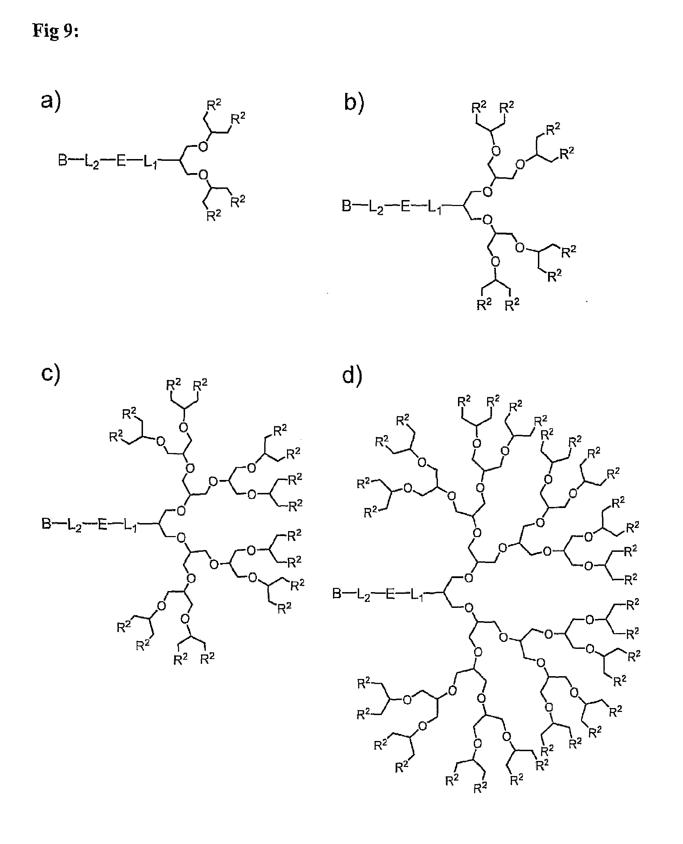

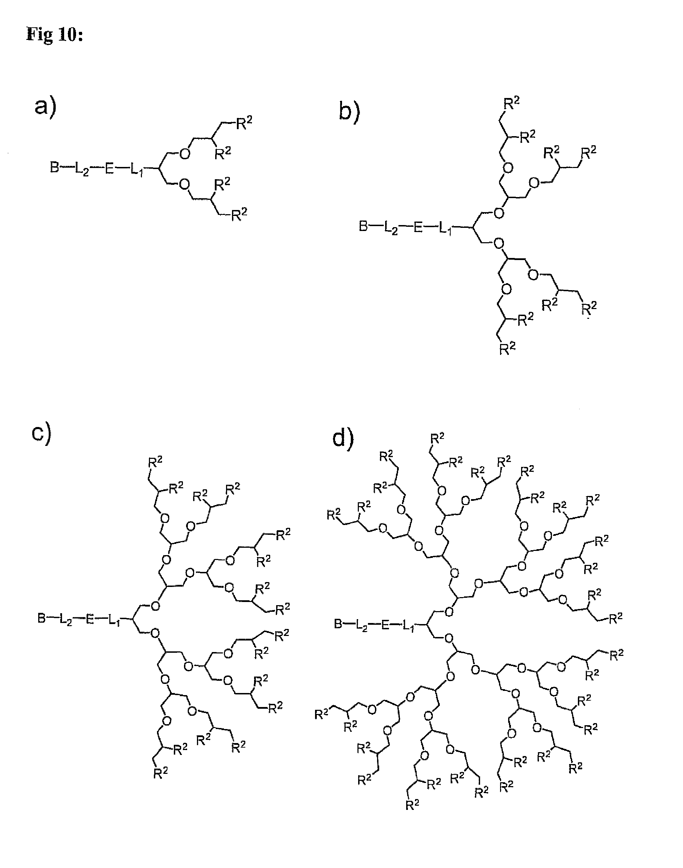

In another preferred embodiment one effector molecule (E) is covalently linked to the functional group R¹ of the core unit of one or more polyether polyol dendron moieties (D),
wherein one biological targeting molecule (B) may be covalently linked to the to the effector molecule (E), and
wherein independently the groups R¹ and R², the core unit and the building block units of the polyether polyol dendron moiety and the integer n may be the same or different for the first polyether polyol dendron moiety and the further polyether polyol dendron moieties, whereby the integer n in the further dendron moieties may also have a value of 0.
Exemplary representations of such dendron conjugates are pictured in Figures 9 and 10, whereby in these figures examples with only one dendron moiety are pictured, which is however to be understood as non-limiting for the above disclosure.

In cases, where said polyether polyol dendron conjugate comprises more than one dendron moiety (D), the polyether polyol dendron conjugate preferably comprises 2 to 8, more preferably 2 to 4, most preferably 2 dendron moieties (D).

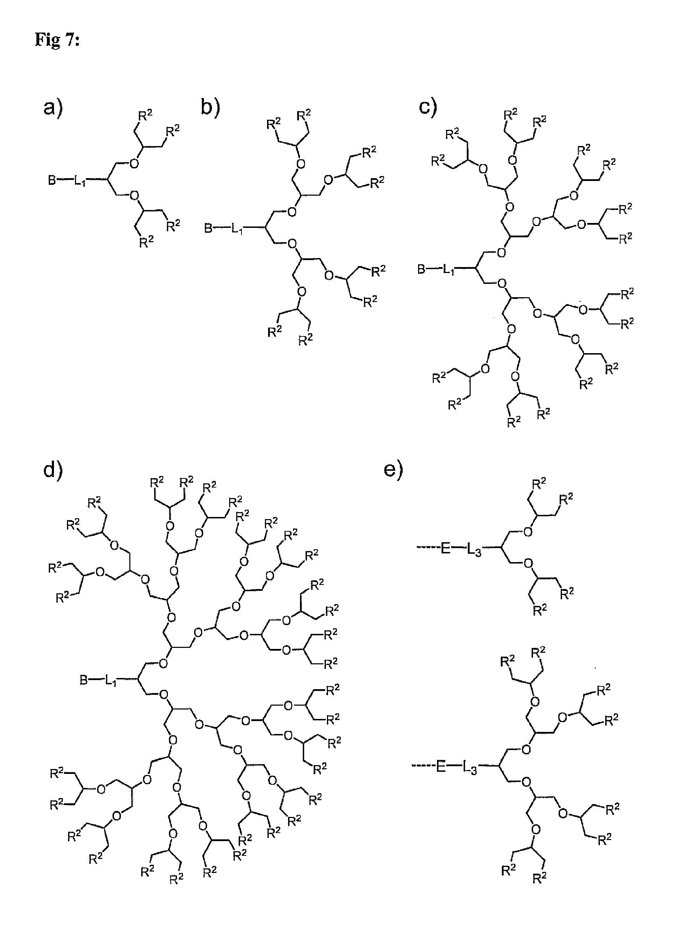

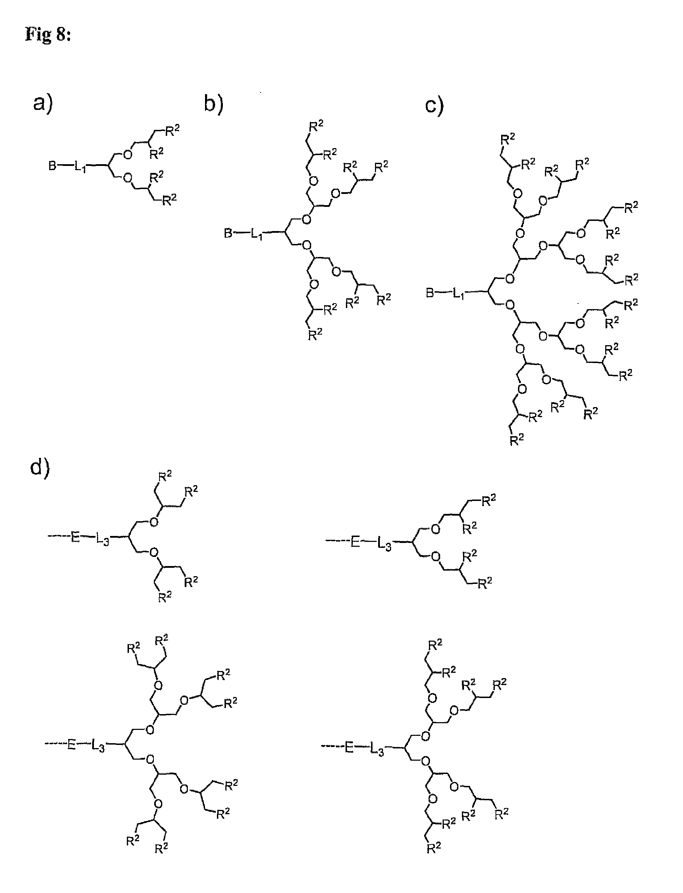

In another preferred embodiment one effector molecule (E) is covalently linked to the functional group R¹ of the core unit of a second polyether polyol dendron moiety (D), wherein the integer n in the second dendron moiety may also have a value of 0,
wherein one or more molecules of the resulting conjugate are covalently linked to one or more of the functional groups R² of one or more of the shell units of a first polyether polyol dendron moiety according to claim 1,
wherein one biological targeting molecule (B) may be covalently linked to the functional group R¹ of the core unit of the second polyether polyol dendron moiety, and
wherein independently the groups R¹ and R², the core unit and the building block units of the polyether polyol dendron moieties and the integer n may be the same or different for the first polyether polyol dendron moiety and the second polyether polyol dendron moiety.
Exemplary representations of such dendron conjugates are pictured in Figures 7 and 8.

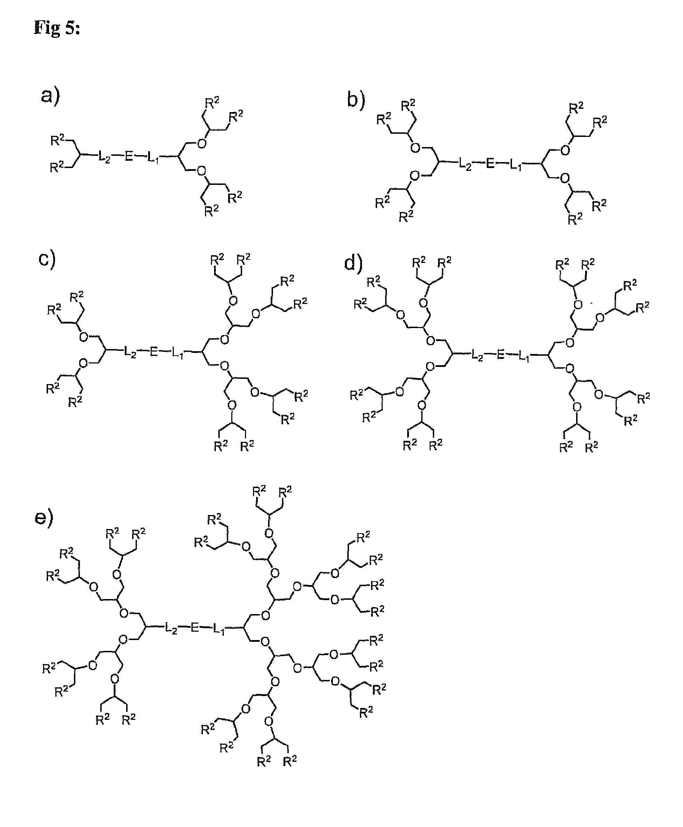

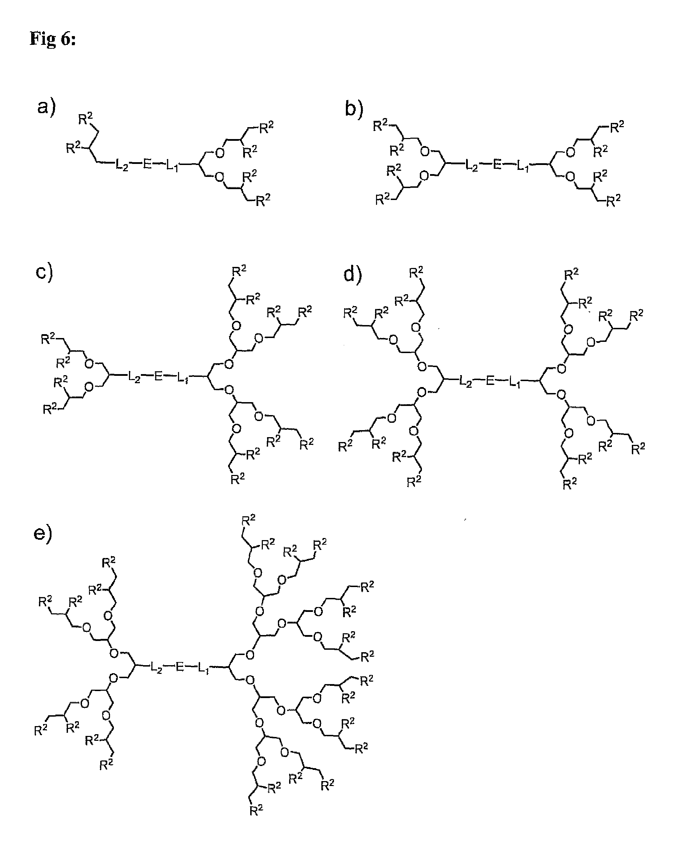

In another preferred embodiment more than one polyether polyol dendron moieties (D) according to claim 1 are covalently linked to one effector molecule (E) via their respective groups R¹ of the core units,
wherein one or more biological targeting molecules (B) may be covalently linked to the one or more of the functional groups R² of one or more of the shell units of one or more polyether polyol dendron moieties (D), and
wherein independently the groups R¹ and R², the core unit and the building block units of the polyether polyol dendron moiety and the integer n may be the same or different for each of the polyether polyol dendron moieties (D), and, wherein the integer n in all but one of the dendron moieties may also have a value of 0.
Exemplary representations of such dendron conjugates are pictured in Figures 5 and 6.

In the above-mentioned cases wherein the dendron conjugate comprises more than one polyether polyol dendron moiety, the core units of further dendron moieties in which the integer n has a value of 0 may be such, that the two hydroxyl groups of the core units of the further dendron moieties may independently be replaced by functional groups R². For an exemplary representation of such dendron conjugates, compare Figures 5 a) and 6 a), in which the integer n has a value of 0 for one of the dendron moieties.

In another more preferred embodiment, for dendron conjugates comprising more than one polyether polyol dendron moiety (D), R¹ is the same for each of the polyether polyol dendron moieties (D).

In another more preferred embodiment, for dendron conjugates comprising more than one polyether polyol dendron moiety (D), the groups R² are the same for each of the polyether polyol dendron moieties (D).

This still means that, in an individual exterior unit, the more than one groups R² may be selected independently from one another, but the selection of R² groups is the same for all of the exterior units.

In another more preferred embodiment, for dendron conjugates comprising more than one polyether polyol dendron moiety (D), the core unit is the same for each of the polyether polyol dendron moieties (D).

In another more preferred embodiment, for dendron conjugates comprising more than one polyether polyol dendron moiety (D), the building block units are the same for each one of the polyether polyol dendron moieties (D).

In another embodiment, for dendrimer conjugates comprising more than one polyether polyol dendron moiety (D), the integer n is different for each of the polyether polyol dendron moieties (D).

In yet another embodiment, for dendrimer conjugates comprising more than one polyether polyol dendron moiety (D), the integer n is the same for each of the polyether polyol dendron moieties (D).

In a further embodiment the polyether polyol dendron moiety (D), the at least one effector molecules (E) and, if present, the biological targeting molecule (B), are covalently linked by linker units (L) independently selected from a direct bond or an aliphatic C₁₋₂₀ hydrocarbon chain,
wherein optionally 1-5 non-consecutive methylene groups may be replaced by a group selected from the group comprising -O-, -S-, -C(O)-, C(O)NH-, -NHC(O)-, -NHC(O)NH-, -C(O)O-, -OC(O)O-, -SO₂-, -O-maleinimide-, -O-succinimide-, triazol, aryl, ethene and ethyne, and wherein the aliphatic C₁₋₂₀ hydrocarbon chain may optionally be substituted with one or more groups selected from the group comprising OH, COOH, O-C₁₋₆-alkyl or phenyl.

Preferably, the linker units (L) are independently selected from aliphatic C₁₋₁₂ hydrocarbon chains, wherein at least one of the carbon atoms is replaced by a group selected from the group comprising -O-, -S- and -C(O)NH- and the hydrocarbon chain may optionally be substituted with OH

A suitable polyether polyol dendron conjugate precursor for the production of polyether polyol dendron conjugates according to the present invention is a precursor comprising a polyether polyol dendron moiety (D) according to the present invention, at least one effector molecule (E) according to the present invention covalently linked to the functional group R¹ of the core unit and/or to one or more of the hydroxyl groups of the shell units of the polyether polyol dendron moiety,
wherein the core unit furthermore comprises a reactive group X for conjugation to a biological targeting molecule (B), or to a second polyether polyol dendron.

Another suitable polyether polyol dendron conjugate precursor for the production of polyether polyol dendron conjugates according to the present invention is a precursor comprising a polyether polyol dendron moiety (D) according to the present invention, at least one biological targeting molecule (B) covalently linked to the functional group R¹ of the core unit and/or to one or more of the hydroxyl groups of the shell units of the polyether polyol dendron moiety,
wherein the core unit furthermore comprises a reactive group X for conjugation to an effector molecule (E) according to the present invention, or to a second polyether polyol dendron.

Preferably, in the polyether polyol dendron conjugate precursor as described above the reactive group X is selected from the group comprising nitro, amino, hydroxy, thiol, maleimide, maleimideacylamino, pyridinyl-disulfide, vinylsulfone, bromoacetyl, iodoacetyl, bromoacetylamide, iodoacetylamide, isothiocyanate, isocyanate, hydrazine, hydrazide, mixed anhydrides, activated esters, in situ activated esters, carboxylic acid-n-hydroxysuccinimidylester, carboxylic acid p-nitrophenylester, sulfonyl chloride, azide, thiobenzylester, arylborane.

Another embodiment of the present invention is a kit, comprising one or more polyether polyol dendron conjugates or polyether polyol dendron conjugate precursors as disclosed in the present application.

Another embodiment of the present invention is a polyether polyol dendron conjugate according to the present invention for therapeutic or diagnostic purposes.

Another embodiment of the present invention is a polyether polyol dendron conjugate according to the present invention for therapeutic or diagnostic purposes in a disease state or condition selected from the group comprising disease states or conditions related to tumors, tumor metastases, atherosclerosis, inflammation, (preferably rheumatoid arthritis, osteoarthritis), ocular diseases, precanceroses, metaplasia, hyperplasia, and benign lesions (preferably benign prostate hyperplasia).

Other tumors which may be diagnosed according to the present invention are selected from a malignoma of the gastrointestinal or colorectal tract, liver, pancreas, kidney, bladder, thyroid, prostate, endometrium, ovary, testes, melanoma, dysplastic oral mucosa, invasive oral cancer, small cell or non-small cell lung carcinoma; a mammary tumor, including hormone-dependent breast cancer, hormone independent breast cancers; transitional and squamous cell cancers; neurological malignancy including neuroblastoma, gliomas, astrocytomas, osteosarcoma, meningioma; soft tissue sarcoma; hemangioama and an endocrinological tumor, including pituitary adenoma, pheochromocytoma, paraganglioma, a haematological malignancy including lymphoma and leukemia or the metastasis originates from one of above mentioned tumors. Particularly preferred tumors are tumors of the breast, cervix, prostate, testis, wherein the organ/tissue from which the tumor developed is easily accessible from outside the body or by endoscopic means.

The ocular disease may for instance be selected from the group consisting of trachoma, retinopathy of prematurity, diabetic retinopathy, neovascular glaucoma and age-related macular degeneration. Preferred is the diagnosis and therapy of age-related macular degeneration.

Another preferred embodiment of the present invention is a polyether polyol dendron conjugate according to the present invention for therapeutic or diagnostic purposes in a disease state or condition selected from prostate cancer, benign prostate hyperplasia, age-related macula degeneration and breast cancer.

In this context, preferred diagnostic applications are *in vivo* diagnostics, which are conducted by acquiring fluorescence images of regions of the body by illuminating the region of interest with light to excite the fluorescence of a diagnostic effector molecule (E) in the dendron conjugate, and capture images of the fluorescence emission using a detection device.

A Preferred therapeutic application is Photodynamic Therapy (PDT) which is conducted by illuminating the region of interest with light to induce phototoxicity (photosensibilization) of a therapeutic effector molecule (E) in the dendron conjugate and generate as a result local cytotoxic singlet oxygen and/or radicals leading to cell death and therapeutic efficacy.

Another embodiment of the present invention is the synthesis of a polyether polyol dendron moiety (D) according to the present invention, wherein the polyether polyol dendron moiety (D) is built up sequentially from shell to core in a converging synthesis,
wherein two building block units are coupled to the functional groups R² of a core unit or a further building block unit, whereby dendron moiety precursors are obtained, which may again be coupled to the functional groups R² of a core unit or a further building block unit, whereby the process is repeated in order to obtain a dendron moiety with the desired number of generations, thereby doubling the number of exterior R² groups of the growing dendron moiety in each synthesis step.

An example of such a dendron moiety precursor is represented by triglycerol, which is commercially available and in which R¹ = R² = OH.

It is apparent to a person skilled in the art that the synthesis of polyether polyol dendron moieties according to the present invention can also be conducted in such a way that the polyether polyol dendron moiety is built up sequentially from core to shell in a diverging synthesis, whereby for each new generation the R² groups of the building block units are OH-groups each of which is reacted with a further building block unit in which the reactive groups R² may be modified to form new OH groups, whereby this process may be repeated in order to obtain a dendron moiety with the desired number of generations, thereby doubling the number of exterior R² groups of the growing dendron moiety in each synthesis step. In a final step, the exterior building units are attached.

The above-mentioned methods of convergent and divergent synthesis may be combined in such that smaller dendron moiety precursors are synthesized in a diverging synthesis, of which two molecules are then coupled to a core unit or building block unit in accordance with the convergent approach described above in order to obtain a dendron moiety (or precursor) in which the number of exterior R² groups is doubled in comparison to the smaller dendron moiety precursors.

Another embodiment of the present invention is the synthesis of a polyether polyol dendron conjugate according to the present invention by conjugation of a precursor according to the present invention to one or more biological targeting molecules (B) or an effector molecule (E), forming a covalent bond between the a reactive group X and a functional group of the one or more biological targeting molecules (B) or one or more effector molecules (E).
Hereby, the covalent bond is preferably selected from thiol, hydroxy, amine, or histidine.

In the following, the meaning of several expressions used in the context of the present invention is further explained.

The term "dendritically repeating" is used to describe that repeating molecular units are used to build up a polymeric molecule, whereby each of the repeating molecular units comprises a multitude of groups to which further molecules can be linked. The resulting polymeric molecule resembles a branched or dendritic structure, similar to branches or roots of a tree. Figure 11 is a schematic representation of a dendron.

When linked to the shell of the dendron moiety (D), the effector molecules (E) and/or biological targeting molecules (B) are not necessarily linked to a specific group R² of the exterior units, but are spread statistically among the groups R² of the exterior units. A skilled person, who has an understanding of organic chemistry, will understand that the average number of (E) and/or (B) can be adjusted by choosing a proper ratio of (B) to (E) and/or (D) in the manufacturing process of the dendron conjugate.

The polyether polyol dendron conjugates according to the present invention are particularly well-suited for the purposes described herein, due to their high bioavailability, their biocompatibility and their stability against degradation. Fluorescent molecules attached to polyether polyol dendron conjugates according to the present invention exhibit an increased fluorescence quantum yield or singlet oxygen yield, respectively.

More than one effector molecule can be attached to the polyether polyol dendron moieties according to the present invention. Due to the rigid, hyper-branched structure of the polyether polyol dendrons, aggregation of the effector molecules is effectively prevented. Such conjugates of a plurality of effectors may then be coupled to biological targeting molecules. Hereby, the polyether polyol has the effect that the molecular interaction of the effectors is minimized, and the additive efficacy of multiple effector molecules can be exploited. Furthermore, the conjugation of a readily prepared dendritic molecule carrying one or more effectors to biological targeting molecules allows a directed coupling to a predefined position of the biological targeting molecules (e.g., in a protein, at a cysteine by coupling via a maleimide entity). Due to the high hydrophilicity of the dendritic unit, the solubility of the resulting conjugate is not negatively affected.

In diagnostic imaging applications, the higher fluorescence signal may lead to better *in vivo* signal-to-noise ratios and thus an improved detection of lesions, in particular when said lesions located in deeper tissue areas. As a benefit of the higher fluorescence efficacy, lower doses of the conjugates or dye derivatives can be applied to the patient. In photodynamic therapy applications, the higher singlet oxygen yields allow a more effective treatment of lesions (tumors, inflammation) by light irradiation and photodynamic therapy. Lesions can be treated in deeper tissues and larger volumes can be cured.

### Examples

### Synthesis:

### Synthesis of fluorescent dyes:

Examples are published in "Topics in Applied Chemistry: Infrared absorbing dyes" Ed. M. Matsuoka, Plenum, N.Y. 1990, "Topics in Applied Chemistry: The Chemistry and Application of Dyes", Waring et al., Plenum, N.Y., 1990, Cytometry 10:3-10 (1989); Cytometry 11:418-430 (1990); Cytometry 12:723-730 (1991); Bioconjugate Chem. 4:105-11 (1993); Bioconjugate Chem. 7:356-62 (1996); J. Org. Chem. 60: 2391-2395 (1995), Heterocyclic Comm. 1: 427-430 (1995), Chem. Rev. 92: 1197 (1992), WO 96/23525, J. Org. Chem. 57: 4578-4580 (1992), Bioconjugate Chem. 16:1275-128 (2005); WO 00/71162, WO 01/52746, WO 01/52743.

### Synthesis of_photosensitizers:

Examples are published in WO 03/028628, US 2005/0020559, Zheng G et al, J Med Chem 2001, 44, 1540-1559; Li G et al., J. Med. Chem. 20013, 46, 5349-5359; Lunardi CN et al., Curr Org Chem 2005, 9, 813-821; Chen Y et al., Curr Org Chem 2004, 8, 1105-1134.

### Example 1: Synthesis of polyglycerol dendron conjugates with fluorescent cyanine dye chromophore bis-1,1'-(4-sulfobutyl)indotricarbocyanine-5-carboxylic acid, sodium salt

Example 1a: Acetal protection of triglycerol: Triglycerol (60 g, 0.25 mol) is gently heated to obtain a liquid consistency. 2,2-Dimethoxypropane (150 mL, 1.25 mol) is added followed by slow addition of p-toluenesulfonic acid (PTSA) (3.0 g, 25 mmol). The reaction is carried out over night at 30-40 °C. After a half hour stirring a homogeneous solution is obtained. The resulting yellow/orange solution is neutralized by addition of triethylamine (25 mmol) and subsequent stirred for 30 min at room temperature. Then the solvent is evaporated in vacuo and the remaining crude liquid is purified via filtration over silica gel (ethyl acetate/hexane 1:2, 2:1, 6:1) to give the compound [Gn]-OH in 78 % as pale yellow oil. This compound is the first building block [G1.0]-OH for the stepwise synthesis of subsequent [Gn]-OH generations.

Example 1b: [Gn]-OH (1.05 Eq. per Cl), 60 % NaH (2.5 Eq. per OH) in mineral oil, cat. ammount of [15]crown-5 and freshly distilled dry THF are placed in a dry two neck round bottomed flask under Ar atmosphere. After 2-3 h stirring at 40°C 1.0 Eq. methallyl dichloride, cat. ammount of KI and [18]crown-6 are added into solution. The mixture is stirred under reflux for 12 h. After cooling to room temp., the reaction is quenched with distilled water and extracted with dichloromethane. The organic layer is then dried over Na₂SO₄ and solvent is removed under vacuum. Purification of the residue is achieved by HPLC with 2-propanol/*n*-hexane as eluent to give desired product in high yield. [G2.0]-ene-99 % yield, [G3.0]-ene - 94%, [G4.0]-ene - 92%, [G5.0]-ene - 68%.

Example 1c: After the reaction with methallyl chloride (example 1 b) the exomethylene group has to be transferred into the hydroxy group ([Gn]-OH) before further build-up of the next dendron generation. The [Gn]-ene (1.0 Eq.) is dissolved in dry methanol/dichloromethane 1:1 (c = 0.13 mol/L) and cooled to -78 °C. Ozone was bubbled through the solution until it turned blue. After the removal of excess ozone by use of a vigorous stream of oxygen, sodium borohydride (10.0 Eq.) is added. While stirring for 12 h the mixture is allowed to slowly warm to room temperature, then quenched by addition of saturated NH₄Cl solution followed by stirring for 1 h. The phases are separated and the aqueous layer is extracted with CH₂Cl₂. The combined organic phases are washed with water, dried over anhydrous Na₂SO₄ and filtered. Evaporation of the solvent yields the target compound in pure form. [G2.0]-OH - >99%, [G3.0]-OH - >99%, [G4.0]-OH - >99%, [G5.0]-OH - >99%

Example 1d: Synthesis of azide-modified dendron: To a solution of [Gn]-OH (1.0 Eq.) and triethylamine (1.1-1.5 Eq.) in toluene, which is cooled to 0 °C in an ice bath, is added methanesulfonyl chloride (1.5 Eq.). Progress of the reaction was monitored by TLC. After completion, the precipitate is filtrated and mixture concentrated under vacuum to give oil as a final product (100 %). The crude product is used for next step reaction. To a solution of the [Gn]-OMs (1.0 Eq.) in dry DMF, 5.0 Eq. of sodium azide was added. After being stirred for 3h at 120 °C under argon, the excess of NaN₃ is filtered off and DMF is removed under high vacuum by cryodestillation. The crude product is purified *via* filtration over thin layer of silica gel (ethyl acetate/hexane). The [Gn]-N₃ is obtained as light yellow, viscous oil with very high yield over two steps. [G2.0]-N₃ - 96%, [G3.0]-N₃ - >99%, [G4.0]-N₃- 91%, [G5.0]-N₃ - >99%.

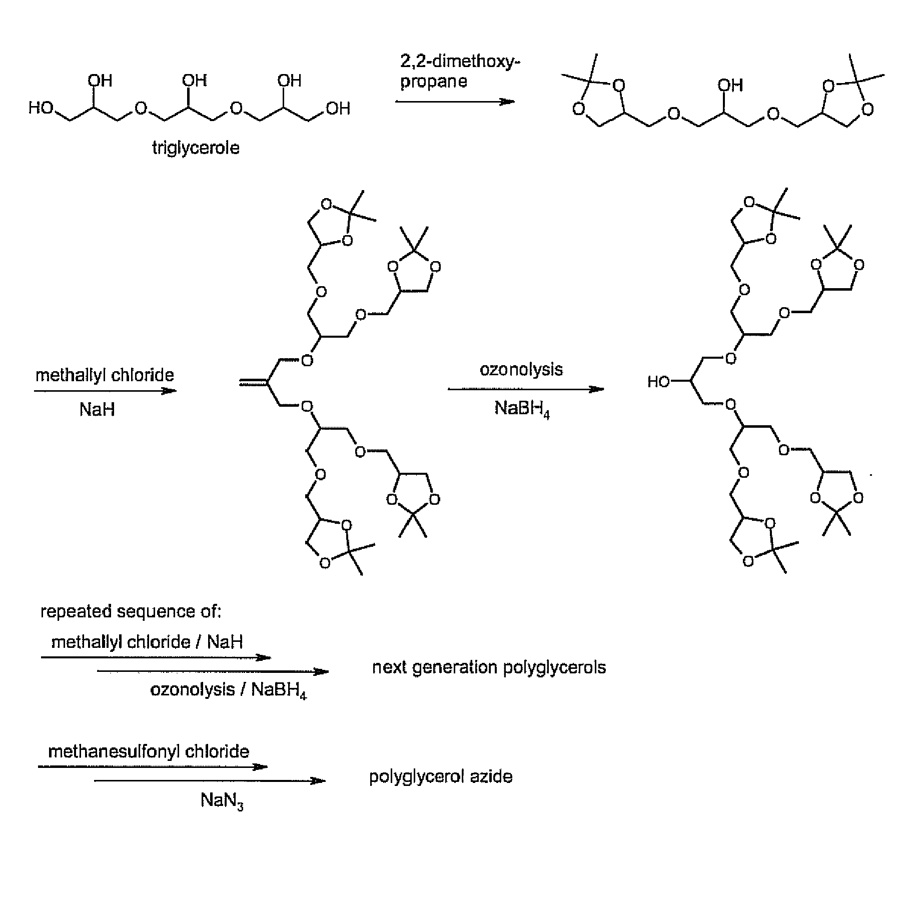

Example 1e: General procedure of Click-coupling of dendron-azides (example 1d) to dendron conjugates with cyanine dye:

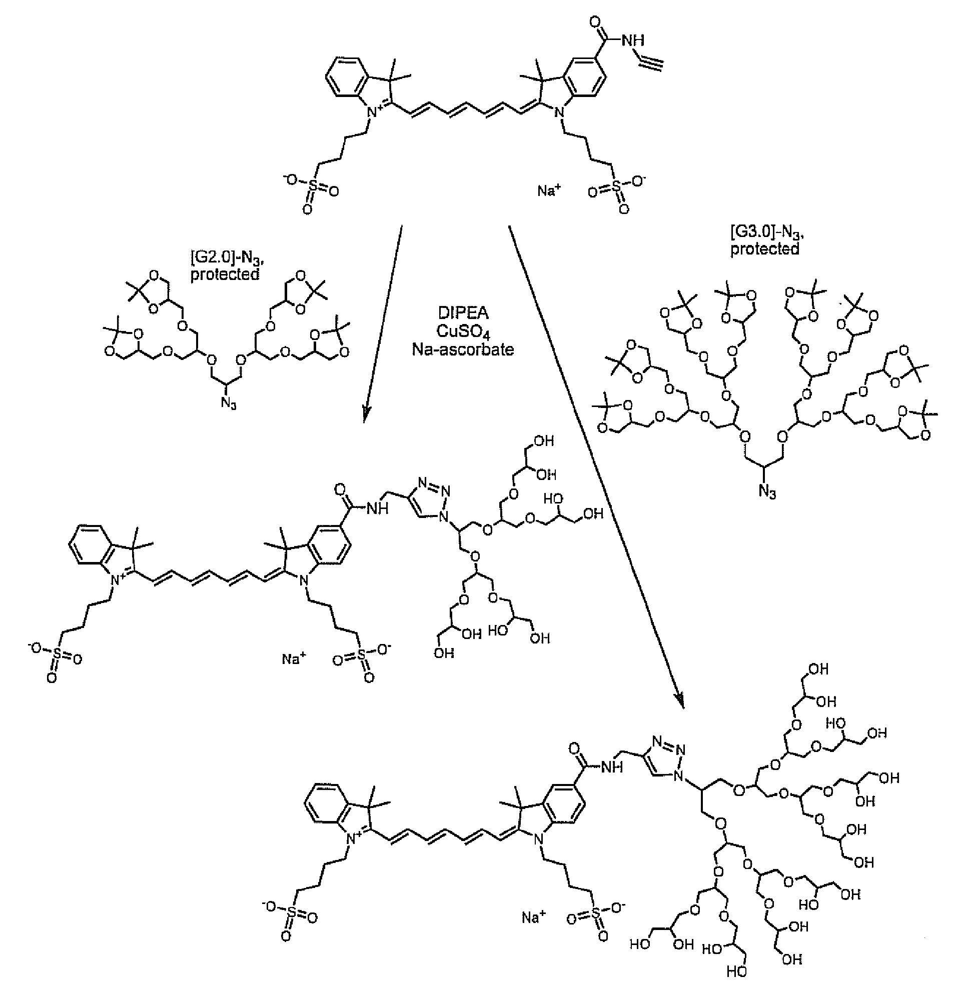

To 1.0 eq. of [Gn]-N₃ and 1.1 eq of bis-1,1'-(4-sulfobutyl)indotricarbocyanine-5-carboxylic acid propargylamide (obtained from 1,1'-(4-sulfobutyl)indotricarbocyanine-5-carboxylic acid, Bioconjugate Chem 12, 2001, 44-50, and propargylamine by common procedures) dissolved in THF is added 0.3 Eq. of DIPEA (diisopropylethyl amine). Next 0.3 Eq. of sodium ascorbate and 15 mol% of copper (II) sulfate pentahydrate is added into reaction mixture. The THF:H₂O mixture has to be 1:1 (v/v). The heterogeneous mixture is stirred vigorously for 48 h at room temperature and concentrated *in vacuo* to dryness. To remove the acetal protecting groups, the residue was dissolved in methanol, acidified with HCl and stirred for 24 h. After evaporation, the crude material is directly purified by reversed phase chromatography (RP-18 Merck Licroprep, water/methanol) to yield the products as blue lyophilisates (yields 67 - 85%).

### Example 2:

Synthesis of polyglycerol dendron conjugates with fluorescent cyanine dye chromophore bis-1,1'-(4-sulfobutyl)benzindotricarbocyanine, sodium salt (indocyanine green derivative).

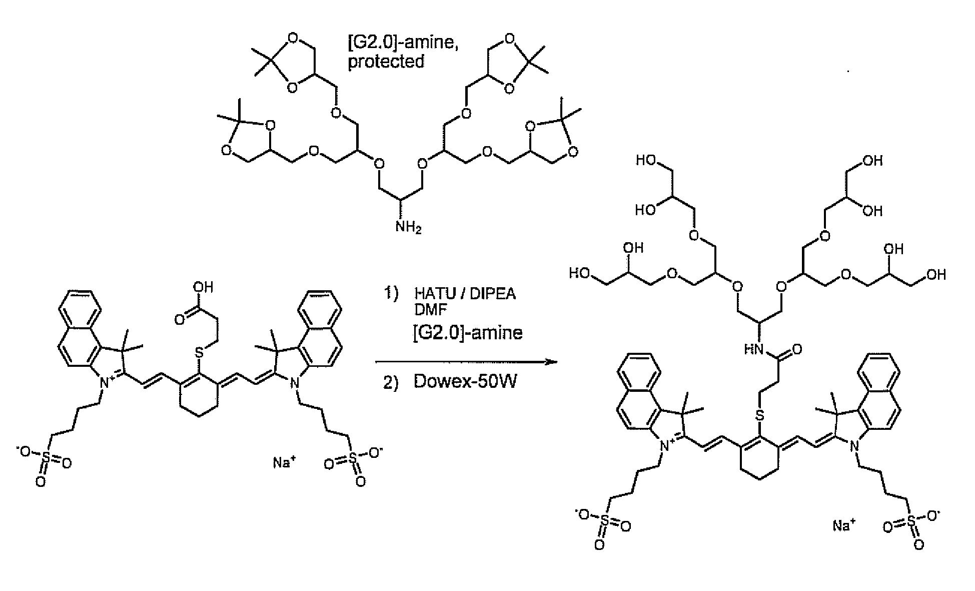

Polyglycerol amines [Gn]-NH₂ are obtained as described in example 1 and according to published procedures for the conversion of azide to amine (Roller S et al., Molecular Diversity 2005, 9: 305-316). Indocyanine green derivative with central cyclohexyl bridge and carboxyethylthio-linker is obtained from commercial IR-820 according to Hilderbrand SA (Bioconjugate Chem. 2005, 16, 1275-128). A solution 25 mg of this derivative (0,027 mmol), 12 mg of HATU (0,032 mmol), 8.5 mg of DIPEA (0,065 mmol) in dry DMF is stirred for 15 min. and treated with a solution of [G2.0]-amine, acetal-protected (38 mg, 0,054 mmol) in DMF. The resulting mixture is stirred at 25°C for 48 h and the product precipitated by addition of diethyl ether. The residue is dissolved in methanol and stirred for 8 h at 50°C with ion exchange resin Dowex 50W to remove the acetal protection groups. After filtering off the Dowex resin, the solution is evaporated to dryness and the product isolated by reversed phase chromatography (RP-18 Merck Licroprep, water/methanol); overall yield 34%.

### Example 3:

### Synthesis ofpolyglycerol dendron conjugates with perylene dye

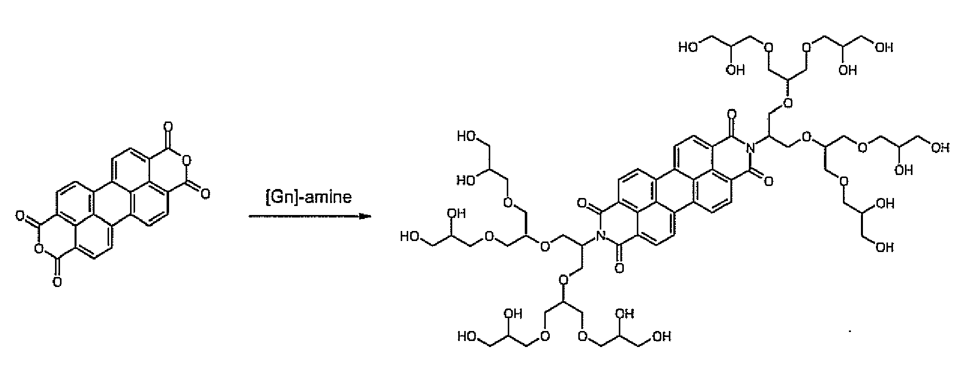

Polyglycerol amines [Gn]-NH₂ are obtained as described in example 1 and according to published procedures for the conversion of azide to amine (Roller S et al., Molecular Diversity 2005, 9: 305-316). 100 mg (0.18 mmol) polygylcerol amine ([G2.0]-amine), 30 mg perylene anhydride (0.08 mmol) and 500 mg imidazole are heated to 140 °C for 3.5 hours, and then cooled to room temperature. The remaining solid is dissolved in water to give a red to purple coloured solution which is dialysed over three days with water (MWC 500) to give 102 mg (92% conversion) of an amorphous dark purple solid. ¹H-NMR (D₂O, 250 MHz) δ (ppm) = 7.943-7.545 (two broad signals, 8H, aromatic), 5.538 (broad signal, 2H, -N-CH-,), 4.331-3.651 (broad signal, 68H, PG-Backbone). ESI-MS 1449.5722 [M+Na]⁺; 736.2811 [M+Na]²⁺_{.}

### Example 4:

### Synthesis and fluorescence quantum yield of polyglycerol dendron conjugates with fluorescent cyanine dye chromophore bis-1,1'-(4-sulfobutyl)indotricarbocyanine-5,5'-dicarboxylic acid, sodium salt

### Example 4a: Synthesis of polyglycerol dendron conjugates with fluorescent cyanine dye chromophore bis-1,1'-(4-sulfobutyl)indotricarbocyanine-5,5'-dicarboxylic acid, sodium salt

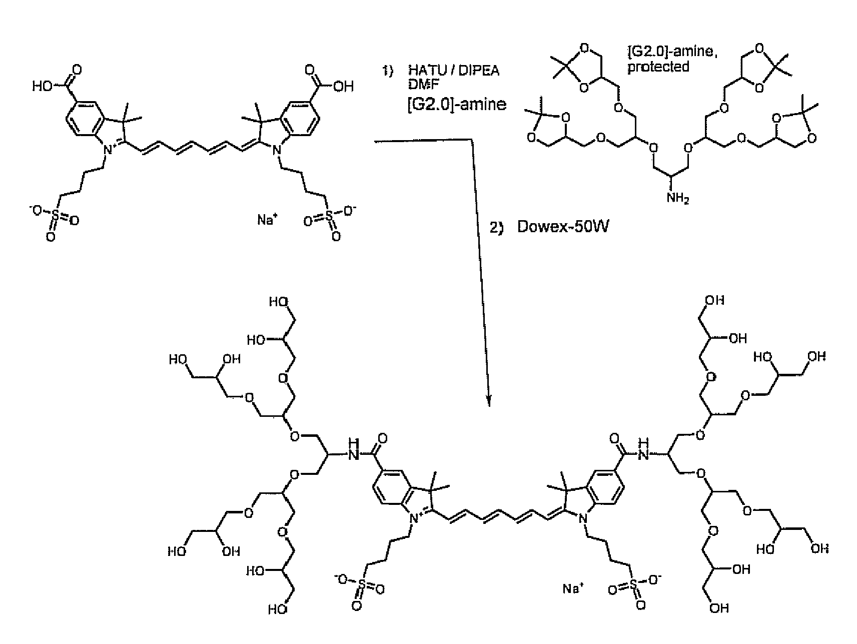

Polyglycerol amines [Gn]-NH₂ are obtained as described in example 1 and according to published procedures for the conversion of azide to amine (Roller S et al., Molecular Diversity 2005, 9: 305-316). Bis-1,1'-(4-sulfobutyl)indotricarbocyanine-5,5'-dicarboxylic acid, sodium salt is synthesized according to Licha et al., Photochem Photobiol 2000, 72, 392-398.

A solution 50 mg bis-1,1'-(4-sulfobutyl)indotricarbocyanine-5,5'-dicarboxylic acid, sodium salt (0.063 mmol), 48 mg of HATU (0.13 mmol), 34 mg of DIPEA (0.26 mmol) in dry DMF is stirred for 15 min. and treated with a solution of [G2,0]-amine, acetal-protected (114 mg, 0.162 mmol) in DMF. The resulting mixture is stirred at 40°C for 72 h and the product precipitated by addition of diethyl ether. The residue is dissolved in methanol and stirred for 8 h at 50°C with ion exchange resin Dowex 50W to remove the acetal protection groups. After filtering off the Dowex resin, the solution is evaporated to dryness and the product isolated by reversed phase chromatography (RP-18 Merck Licroprep, water/methanol); overall yield 54%.

### Example 4b: Fluorescence quantum yield of title compound of example 4a in comparison to precursor dye dendron bis-1,1'-(4-sulfobutyl)indotricarbocyanine-5,5'-dicarboxylic acid, sodium salt

### Fluorescence quantum yields:

- Dendron dye (example 4a):: 0.13 (PBS); 0.18 (MeOH)
- Precursor dye:: 0.07 (PBS); 0.14 (MeOH)

### Example 5:

### Synthesis of polyglycerol dendron conjugate with photosensitizer purpurnimide derived from purpurin-18

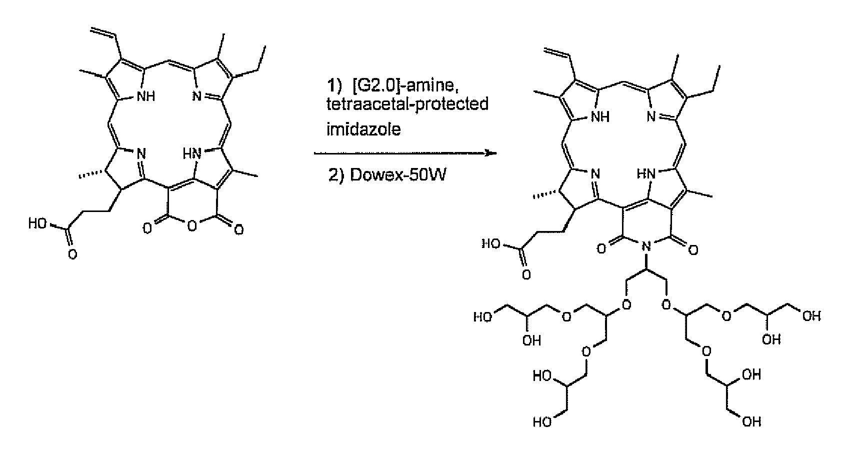

50 mg (0.089 mmol) purpurin-18 (J Med Chem 2001, 44, 1540-1559) and 35 mg polygylcerol amine ([G2.0]-amine, acetal-protected, see example 2) are reacted in presence of imidazole as described in example 3. The mixture is diluted with dichloromethane, washed with brine, and evaporated to dryness. The solid is dissolved in 10 mL methanol, Dowex-50W is added, and the mixture stirred for 12 h at 60°C. After evaporation to approx. 2-3 mL, diethyl ether (20 mL) is added and the resulting precipitate collected by filtration. Purification by reversed phase chromatography (RP-18 Merck Licroprep, water/acetonitrile, incl. 0.01% TFA) yields 24 mg of product (25%) as green solid after lyophilization; MS MALDI 1082, 105.

### Example 6:

### Synthesis of polyglycerol dendron conjugate with photosensitizer 3-formylpyropheophorbide a via reductive amination.

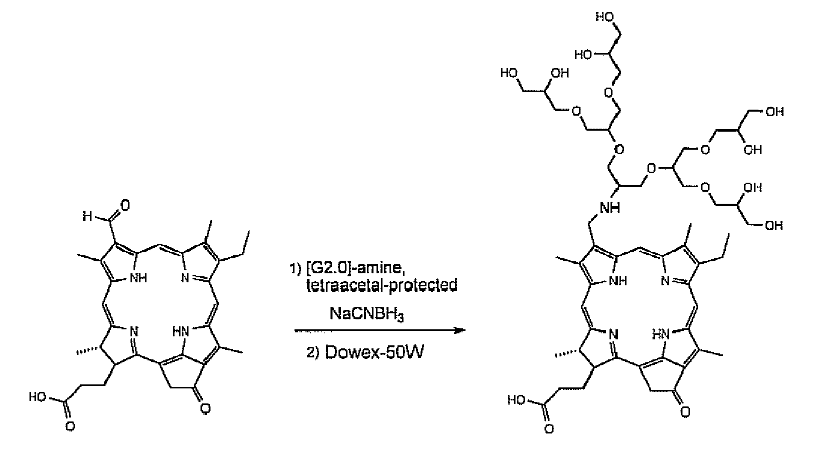

3-Formylpyropheophorbide a is obtained according to Photochem Photobiol 81, 2005, 170-176. To a solution of 25 mg (0.047 mmol) of 3-formylpyropheophorbide a and 50 mg (0.071 mmol) [G2.0]-amine, tetraacetal-protected, in THF is added 17 mg (0.080 mmol) sodium-triacetoxyborohydride and 5.4 mg (0.09 mmol) acetic acid (51 µL of a stock solution of 1 mL in 10 mL THF). The mixture is stirred for 48 h at room temperature, diluted with water and lyophilized. The residue is dissolved in HCl/methanol and stirred 18 h to remove the acetal groups. After adjustment of the pH to 7 with 0.1% NaOH the solution is lyophilized. The resulting solid is purified by HPLC (reversed phase, acetonitrile/water +0.01 % TFA) yielding 36 mg product (72%); MS MALDI 1057.

### Example 7:

### Synthesis of polyglycerol dendron conjugate derived from aminooxepanol with two and four fluorescent cyanine dye chromophores bis-1,1'-(4-sulfobutyl)indotricarbocyanine, sodium salt

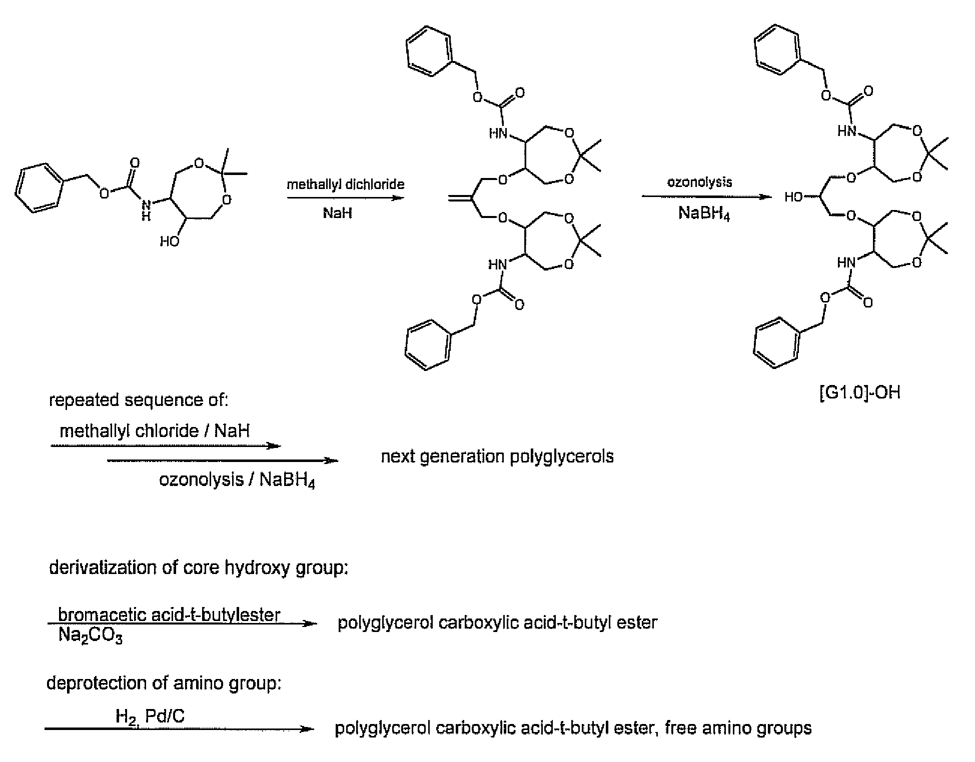

Example 7a: 3 g (10 mmol) of (±)-anti-benzyl-6-hydroxy-2,2-dimethyl-1,3-dioxepan-5-yl carbamate (Tetrahedron Asymmetry 17, 2006, 3128-3134), 60 % NaH (1 g, 25 mmol) in mineral oil, catalytic amount of [15]crown-5 and freshly distilled dry THF are placed in a dry two neck round bottomed flask under Ar atmosphere. After 3 h stirring at 40 °C 1.0 Eq. methallyl dichloride, catalytic amount of KI and [18]crown-6 are added into the mixture. The mixture is stirred under reflux for 12 h. After cooling to room temp., the reaction is quenched with distilled water and extracted with dichloromethane. The organic layer is then dried over Na₂SO₄ and solvent is removed under vacuum. Purification of the residue is achieved by Flash chromatography (silica gel; dichloromethane/methanol) to give the product in 82% yield.

Example 7b: After the reaction with metallyl chloride (example 1b) the exomethylene group has to be transferred into the hydroxy group ([Gn]-OH) before further build-up of the next dendron generation. The reaction path is carried out as described in example 1c giving products in high yield: [G1.0]-OH - 91 %, [G2.0] - 86%.

Example 7c: Synthesis of COOH modified dendrons: The core OH-group is derivatized with bromoacetic acid-t-butyl ester. 1.5 mmol of Gn-OH (1 Eq.) in 40 mL toluene / 4 mL THF are mixed with 100 mg of tetrabutylammoniumsulfate and 30 mL of 32% sodium hydroxide solution, 0.54 g (3 mmol) of bromoacetic acid-tertbutylester is added within 1 h and the resulting mixture stirred for 18 h at room temperature. The organic phase is separated, and the aqueous phase extracted with dichloromethane. The combined organic phases are washed with NaCl solution, dried on sodium sulfate and concentrated by evaporation. Chromatographic purification (silica gel; hexane/ethyl acetate) gives the product as palebrownish oils; [G1.0]-OCH₂COOtBu - 65%, [G2.0]OCH₂COOtBu - 45%.

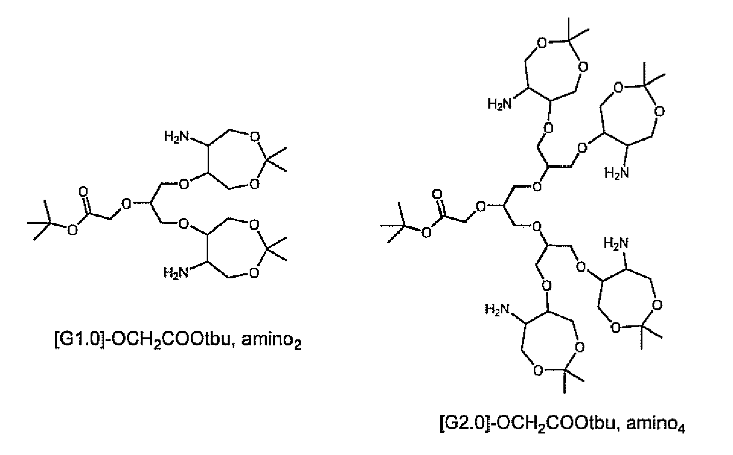

Example 7d: Synthesis of carboxy-modified dendrons with free amino groups by hydrogenation of Cbz protecting group: 0.5 g of Gn-OCH₂COOtBu are dissolved in methanol. After addition of 0.1 g 10%Pd/C-catalyst the mixture is hydrogenated under a H₂-balloon for 18 h. The catalyst is removed by filtration and the solution evaporated to dryness yielding the products as light brownish solids: [G1.0]-OCH₂COOtBu/amino₂ - 98%, [G2.0]-OCH₂COOtBu/amino₄ - 96%.

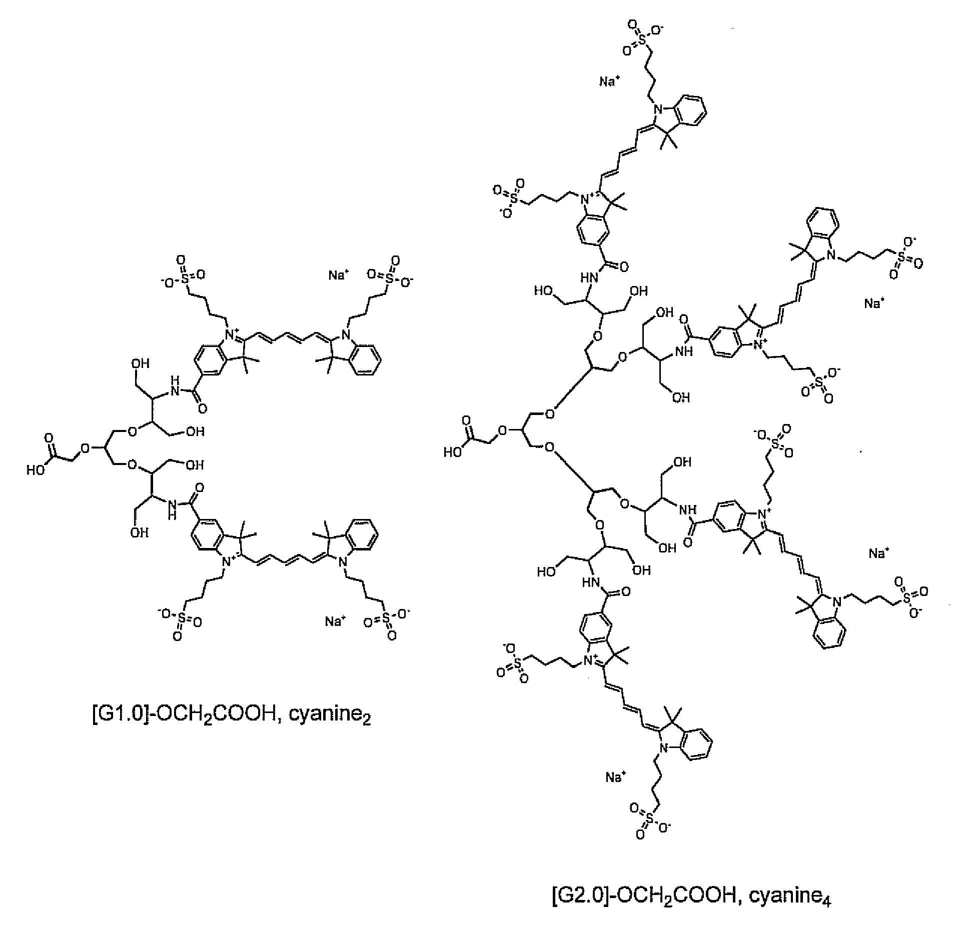

Example 7e: Conjugation of [G1,0]-OCH₂COOtBu/amino₂ and [G2.0]-OCH₂COOtBu/amino₄ (both acetal-protected) with cyanine dye 1,1'-(4-sulfobutyl)indotricarbocyanine-5-carboxylic acid (Bioconjugate Chem 12, 2001, 44-50): A solution of cyanine dye (100 mg, 0.14 mmol) in DMF is treated with 63 mg HATU (0.17 mmol) and 75 mg DIPEA (0.42 mol) and stirred for 30 min at room temperature. To this activated dye is added 0.05 mmol) of [G1.0]-OCH₂COOtBu/amino₂ or 0.02 mmol [G1.0]-OCH₂COOtBu/amino₄, respectively. The reaction is allowed to stir for 5 h at room temperature and 18 h at 40°C. The product is isolated as crude material by precipitation with diethyl ether, and the residue is stirred in 10 mL dichloromethane containing 2 mL trifluoroacetic acid to obtain the free carboxylic acid and free OH-groups. Chromatographic purification is achieved by reversed phase chromatography (RP-18 Merck Licroprep, water/methanol, incl. 0.01% TFA) yielding the products as green solid after lyophilization; yields [G1.0]-OCH₂COOH/ cyanine₂ - 55%; [G2.0]-OCH₂COOH/ cyanine₄ - 34%.

### Example 8:

### Synthesis of polyglycerol dendron conjugate derived from aminooxepanol with two photosensitizer molecules pyropheophorbide a.

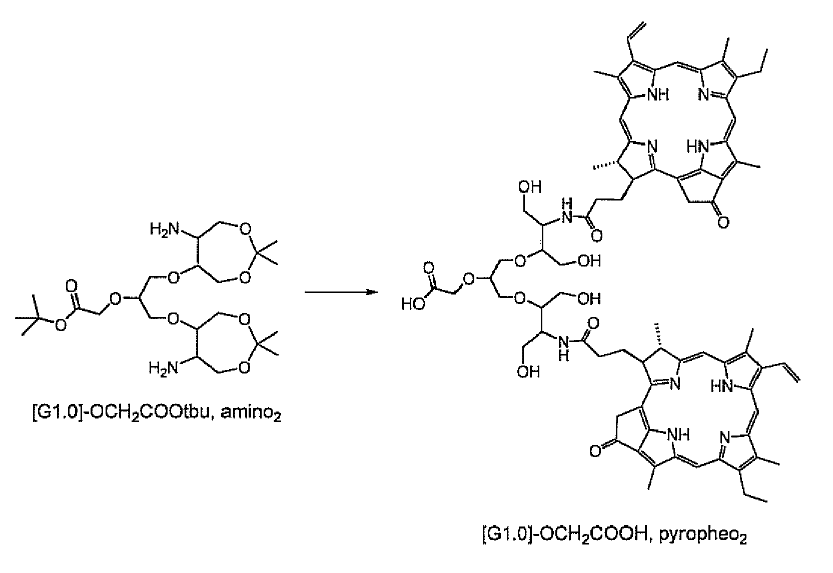

Conjugation of [G1.0]-OCH₂COOtBu/amino₂ (acetal-protected; example 7d) with pyropheophorbide a: A solution of pyropheophorbide a (100 mg, 0.14 mmol) in DMF is treated with 63 mg HATU (0.17 mmol) and 75 mg DIPEA (0.42 mol) and stirred for 30 min at room temperature. To this activated dye is added 25 mg (0.05 mmol) of [G1.0]-OCH₂COOtBu/amino₂ and the mixture is stirred for 24 h at 40°C. After addition of hexane:diethyl ether (3:1) the precipitate is collected by centrifugation, then redissolved in dichloromethane/TFA (3:1), stirred for 3 h and evaporated to dryness. This residue is finally stirred in 10 mL HCl/methanol for 24 h at 50°C. Evaporation to 5 mL and precipitation with diethyl ether afforded crude product, which is purified by flash chromatography (silica gel; ethylacetate/hexane); overall yield 44 mg (64%) of a green solid; MALDI MS 1390, 1412.

### Example 9:

### Synthesis of polyglycerol dendron conjugate derived from aminooxepanol with two photosensitizer molecules pyropheophorbide a-dendron of example 6.

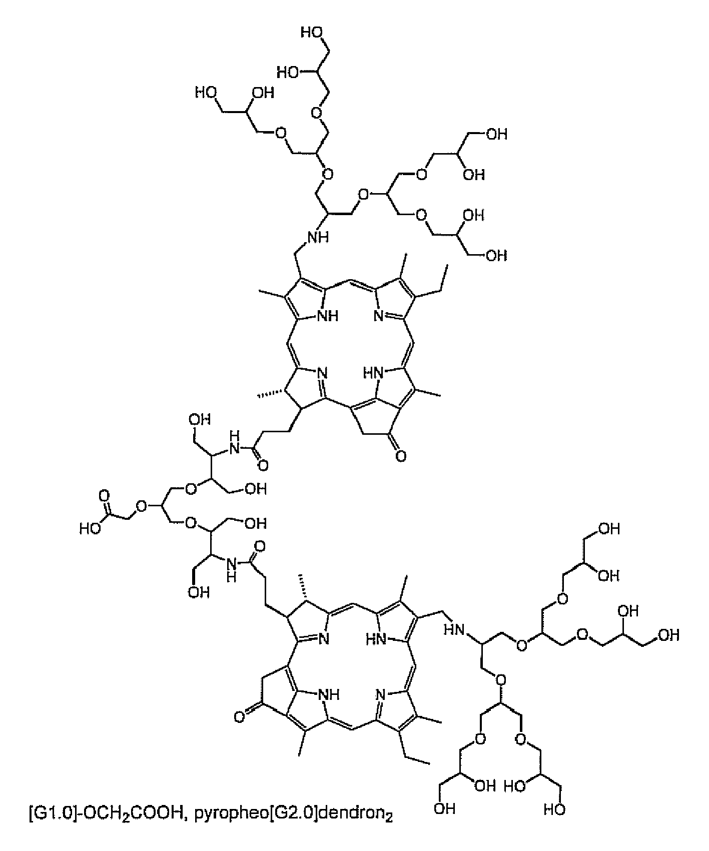

Conjugation of [G1.0]-OCH₂COOtBu/amino₂ (acetal-protected; example 7d) with title compound of example 6: The reaction is carried out similar to the procedure described in example 8, except that the first activation step with HATU is performed at 0°C. The final isolation of product [G1.0]-OCH₂COOH-pyropheo[G2.0]dendron₂ is achieved by HPLC (reversed phase, acetonitrile/water +0.01% TFA), MS MALDI 2435, 2456, 2478.

### Example 10:

Conjugation of polyglycerol dendron conjugate derived from aminooxepanol with [G1.0]-OCH₂COOH-pyrapheo[G2.0]dendron₂ (example 9) with IgG antibody and bovine serum albumine.

Example 10a: Title compound of example 9 (20 mg, 8.2 µmol) is dissolved in dry DMF. To this solution is added 8.3 mg (0.04 mmol) DCC and 9.2 mg (0.08 mmol) N-hydroxysuccinimide. The mixture is stirred for 5 h at 40°C, poured into a centrifugation tube containing diethyl ether, and the resulting precipitate collected by centrifugation. After 3 circles of precipitation, 25 mg of crude N-hydroxysuccinimidyl ester of example 9 are obtained and directly used for conjugation.

Example 10b: Conjugation with IgG antibody: To a solution of 1 mg antibody (IgG from bovine serum; Sigma, > 95%, salt free, powder) in 0.5 mg of phosphate-buffered saline (PBS, pH 7.4) is given 0.067 mmol (32 µL) of a solution of 0.5 mg of N-hydroxysuccinimidyl ester of example 9 in 0.1 mL water. The mixture is shaken at 25°C for 24 h in the dark. Purification is achieved by filtration via a NAP10 column (Pharmacia) using PBS as eluent.
As control conjugate, pyropheophorbide a -NHS-ester is used for conjugation with IgG giving IgG-pyropheo conjugate.

Example 10c: Conjugation with bovine serum albumine (BSA): To a solution of 1 mg BSA (Sigma, fraction V, >98%, powder) in 0.5 mg of phosphate-buffered saline (PBS, pH 7.4) is given 0.148 mmol (70 µL) of a solution of 0.5 mg of N-hydroxysuccinimidyl ester of example 9 in 0.1 mL water. The mixture is shaken at 25°C for 24 h in the dark. Purification is achieved by filtration via a NAP 1 0 column (Pharmacia) using PB S as eluent.
As control conjugate, pyropheophorbide a -NHS-ester is used for conjugation with BSA giving BSA-pyropheo conjugate.

Example 10d: Investigation of solubilities in PBS by observation of precipitates from the solutions obtained in examples 10b and 10c:

| | |
|---|---|
| IgG - [G1.0]-OCH2COOH-pyropheo[G2.0]dendron₂ | no precipitates up to 72 h |
| IgG - pyropheo | precipates after 15 min storage |
| BSA - [G1.0]-OCH₂COOH-pyropheo[G2.0]dendron₂ | no precipitates up to 72 h |
| BSA-pyropheo | immediate precipates |

### Example 11:

### Synthesis of a polyglycerol-cyanine conjugate with a peptide

Example 11a: Modification of hexyl-bridged meso-chloro bis-1,1'-(4-sulfobutyl)-indotricarbocyanine-5-carboxylic acid, sodium salt thiol derivative [G3.0]-O(CH₂)₃-SH

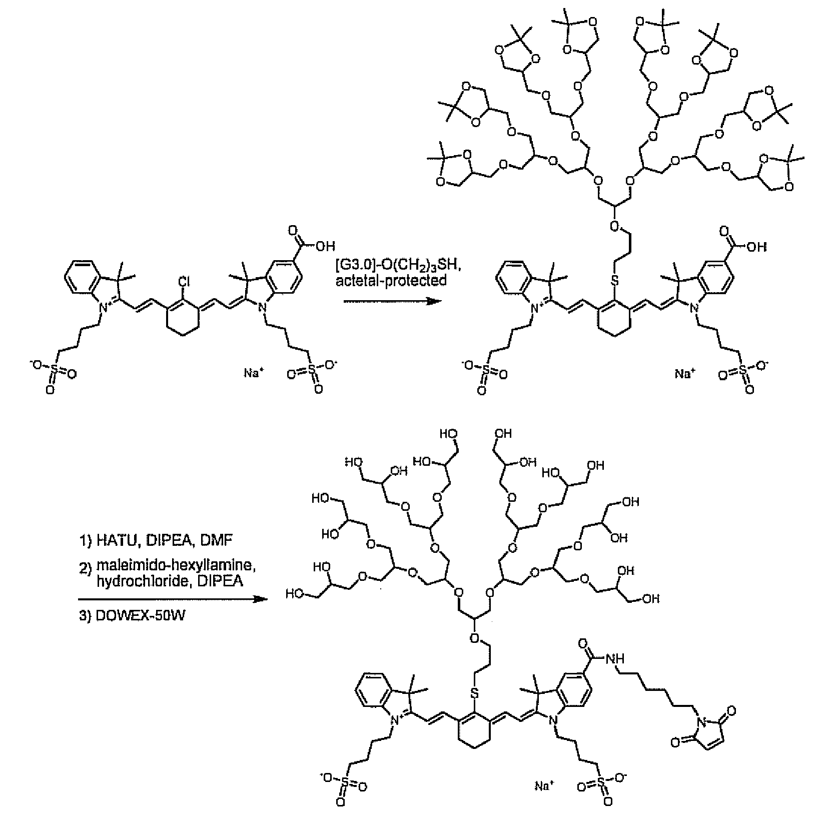

The cyanine dye is synthesized according to known procedures in the literature. [G3.0]-O(CH₂)₃-SH, acetal-protected, is obtained from [G3.0]-OH, acetal-protected, by reaction of the hydroxy group with allyl bromide, followed by modification of the allyl double bond by way of radicalic thioacetyl addition reaction and removal of protecting groups with DOWEX-50W. Reaction of [G3.0]-O(CH₂)₃-SH, acetal-protected, with cyanine dye is accomplished according to Bioconjugate Chem. 2005, 1.6, 1275-128. The compound is obtained after reversed phase chromatography (RP-18 Merck Licroprep, water/methanol, incl. 0.01% TFA) in high purity; yield 82%.

Example 11b: Modification with maleimide linker: A solution 45 mg of this derivative (0.023 mmol), 10 mg of HATU (0.026 mmol), 6.8 mg of DIPEA (0.065 mmol) in dry DMF is stirred for 15 min. and treated with a solution maleimidohexylamine-hydrochloride (15 mg, 0.065 mmol) in DMF. The resulting mixture is stirred at 25°C for 18 h and the product precipitated by addition of diethyl ether. Purification is done by reversed phase chromatography (RP-18 Merck Licroprep, water/acetonitrile) yielding 24 mg (49%) after lyophilization. This intermediate product is dissolved in 3 mL PBS and to this solution is added TAT-binding peptide H-Gly-Arg-Lys-Lys-Arg-Arg-Gln-Arg-Arg-Arg-Gly-Cys-CONH2 (3 *TFA salt; 20 mg; 0.011 mmol). The mixture is incubated at 25°C for 4 h, lyophilized and then dissolved in distilled water for HPLC purification (water/acetonitrile, +0.1% TFA), yielding 15 mg product; MALDI MS: 3691, 3693, 3715

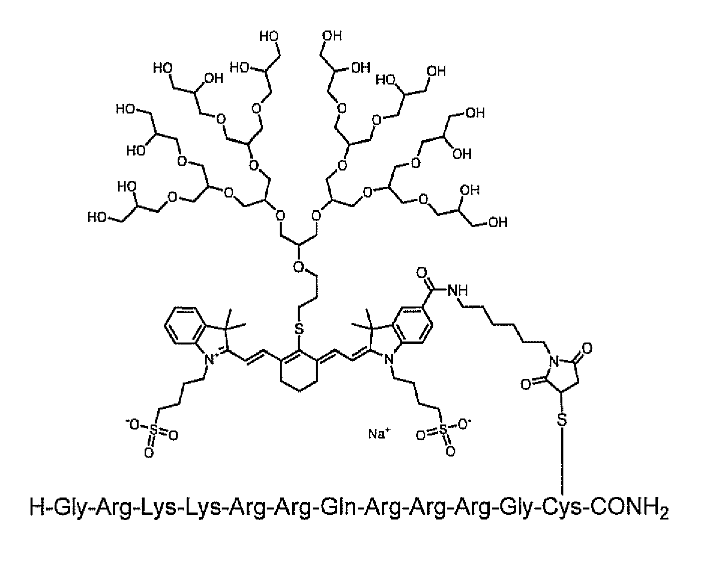

### Brief description of the figures

It is to be understood that the Figures comprised herein are not intended to limit the scope of the present application. The shown core units, building block units, exterior units, groups R¹ and R², as well as the connectivities of the subunits and the number of dendron generations are merely representative Examples to facilitate the understanding of the present invention.
Figure 1 is a schematic representation of specific polyether polyol (here polyglycerol) dendron moieties with a) 1, b) 2, c) 3 and d) 4 dendron generations. The shown dendrons are perfect, as they all comprise the maximum number of building block / exterior units possible. The exterior units shown herein are structurally derived from glycerol and are connected via a hydroxyl group in 2-position.
Figure 2 is a schematic representation of specific polyether polyol (here polyglycerol) dendron moieties with a) 1, b) 2, c) 3 and d) 4 dendron generations. The shown dendrons are perfect, as they all comprise the maximum number of building block / exterior units possible. The exterior units shown herein are derived from glycerol and are connected via a hydroxyl group in 1-position.
Figure 3 is a schematic representation of specific polyether polyol (here polyglycerol) dendron conjugates with a) 1, b) 2, c) 3 and d) 4 dendron generations. In each case, an optical effector molecule (E) is connected to the core unit via a linker molecule (L; here: L₁). The exterior units shown herein are derived from glycerol and are connected via a hydroxyl group in 2-position.
Figure 4 is analogous to Figure 3, whereby the exterior units are connected via the hydroxyl group in 1-position.
Figure 5 is a schematic representation of specific polyether polyol (here polyglycerol) dendron conjugates comprising two polyether polyol (here polyglycerol) dendron moieties with a) 0 and 1, b) 1 and 1, c) 1 and 2, d) 2 and 2 and e) 2 and 3 dendron generations respectively. In each case, an optical effector molecule (E) is connected to the respective core units via a linker molecules (L; here: L₂ and L₁). The exterior units shown herein are derived from glycerol and are connected via a hydroxyl group in 2-position.
Figure 6 is analogous to Figure 5, whereby the exterior units are connected via the hydroxyl group in 1-position.
Figure 7 is a schematic representation of specific polyether polyol (here polyglycerol) dendron conjugates with a) 1, b) 2, c) 3 and d) 4 dendron generations. In each case, a biological targeting molecule (B) is connected to the core unit via a linker molecule (L; here: L₁). The exterior units shown herein are derived from glycerol and are connected via a hydroxyl group in 2-position. The functional groups R² shown in a) to d) may independently be replaced by optical effector molecules (E) or by dendron moieties comprising an optical effector molecule (E), two possible examples of which are shown in e), wherein (E) is connected via a linker group (L; here L₃).
Figure 8 is a schematic representation of specific polyether polyol (here polyglycerol) dendron conjugates with a) 1, b) 2 and c) 3 dendron generations. In each case, a biological targeting molecule (B) is connected to the core unit via a linker molecule (L; here: L₁). The exterior units shown herein are derived from glycerol and are connected via a hydroxyl group in 1-positions. The functional groups R² shown in a) to c) may independently be replaced by optical effector molecules (E) or by dendron moieties comprising an optical effector molecule (E), four possible examples of which are shown in e), wherein (E) is connected via a linker group (L; here L₃).
Figure 9 is a schematic representation of specific polyether polyol (here polyglycerol) dendron conjugates with a) 1, b) 2, c) 3 and d) 4 dendron generations. In each case, a biological targeting molecule (B) and an optical effector molecule (E) are connected sequentially to the core unit via linker molecules (L; here: L₁ and L₂). The exterior units shown herein are derived from glycerol and are connected via a hydroxyl group in 2-position.
Figure 10 is analogous to Figure 9, whereby the exterior units are connected via a hydroxyl group in 1-position.
Figure 11 is a schematic representation of a) a perfect polyether polyol and b) a non-perfect polyether polyol
Figure 12 is a schematic representation of exemplary dyes which may be used in accordance with the present invention; a) indocyanine green; b) derivatives of indocyanine green in accordance with the present invention.

## Claims

1. A polyether polyol dendron conjugate, comprising
a) a polyether polyol dendron moiety (D),
b) at least one effector molecule (E) selected from the group of fluorescent dyes or photosensitizers, or a radioactive effector molecule, selected from the group of radiometal complexes or radiometal chelates,
wherein the polyether polyol dendron moiety (D) consists of a core unit comprising a C₃-C₅ hydrocarbon backbone, at least one functional group R¹, and furthermore having two hydroxyl groups to each of which n = 1-10 generations of dendritically repeating building block units may be covalently linked, whereby the building block units furthermore comprise a C₃-C₅ hydrocarbon backbone and two hydroxyl groups,
wherein the hydroxyl groups of the outermost generation of building block units (exterior units or shell), are replaced by one or more independently selected functional groups R²,
wherein the building block units may all be of the same type or may be selected from more than one type of different units,
wherein the exterior units may all be of the same type or may be selected from more than one type of different units, and
wherein the dendron conjugate exhibits a fluorescence quantum yield in aqueous media of at least 5% in case (E) is a fluorescent dye and a singlet oxygen quantum yield in aqueous media of at least 30% in case (E) is a photosensitizer.

2. A polyether polyol dendron conjugate according to any of the preceding claims, wherein the polyether polyol dendron moiety (D) is a monodisperse polyether polyol dendron.

3. A polyether polyol dendron conjugate according to any of the preceding claims, wherein the at least one functional groups R¹ are independently selected from the group comprising -H, -OH, -OSO₃H, -OSO₃Na, NH₂, -N₃, -COOH, -SH, -SO₃⁻, -C≡C, -C₁₋₂₀-alkyl, -CONH-C₁₋₂₀-alkyl, -NHC(O)-C₁₋₂₀-alkyl and -O-C₁₋₂₀-alkyl,
wherein the C₁₋₂₀-alkyl group is a branched or linear alkyl group in which one or more non-consecutive methylene groups may be replaced by a group selected from the group comprising O, S, NH, C(O)NH, SO₂, SO, aryl, ethene or ethyne, and wherein said alkyl is substituted with at least one groups selected from the group comprising -OH, -OSO₃H, -OSO₃Na, -NH₂, -N₃, -COOH, -SH, -SO₃⁻, -C≡C.

4. A polyether polyol dendron conjugate according to any of the preceding claims, wherein the functional groups R² are independently selected from the group comprising -OH, -OSO₃H, -OSO₃Na, -NH₂, -N₃, -COOH, -SH, -SO₃⁻, -C≡C, -C₁₋₂₀-alkyl, -CONH-C₁₋₂₀-alkyl, -NHC(O)-C₁₋₂₀-alkyl and -O-C₁₋₂₀-alkyl, wherein the C₁₋₂₀-alkyl group is a branched or linear alkyl group in which one or more non-consecutive methylene groups may be replaced by a group selected from the group comprising O, S, NH, C(O)NH, SO₂, SO, aryl, ethene or ethyne, and wherein said alkyl is substituted with at least one groups selected from the group comprising -OH, -OSO₃H, -OSO₃Na, NH₂, -N₃, -COOH, -SH, -SO₃⁻, -C≡C.

5. A polyether polyol dendron conjugate according to any of the preceding claims, wherein the core unit, the building block units and the exterior units are structurally derived from glycerol, whereby in the core unit one of the OH groups is replaced by a group R¹ according to claim 3, and whereby in the exterior units two of the OH groups are replaced by independently selected groups R² according to claim 4.

6. A polyether polyol dendron conjugate according to any of the preceding claims, wherein at least one of the effector molecules (E) is an optical effector molecule with diagnostic function, comprising a fluorescent dye with a fluorescence emission in the UV/visible (400-800 nm) or near-infrared (700-1000 nm) spectral range.

7. A polyether polyol dendron conjugate according to any of the preceding claims, wherein at least one of the effector molecules (E) is an optical effector molecule with therapeutic function, comprising a photosensitizer with phototherapeutic efficacy after excitation in the UV/visible (400-800 nm) or near-infrared (700-1000 nm) spectral range.

8. A polyether polyol dendron conjugate according to any of the preceding claims, wherein at least one of the effector molecules (E) is a radiolabeled complex comprising a chelate of a radionuclide and a chelating agent selected from tetraazacyclododecane chelates and makrocyclic or open-chain aminocarboxylic acids.

9. A polyether polyol dendron conjugate according to any of the preceding claims, wherein the polyether polyol dendron conjugate furthermore comprises c) one or more biological targeting molecules (B).

10. A polyether polyol dendron conjugate according to any of the preceding claims, wherein the polyether polyol dendron conjugate furthermore comprises c) one or more biological targeting molecules (B), independently selected from a peptide, a peptidomimetic molecule, an antibody or a fragment thereof, an antibody mimetic, a protein, an oligonucleotide, a peptide-oligonucleotide, a carbohydrate.

11. A polyether polyol dendron conjugate according to any of claims 1 to 10, wherein the at least one effector molecule (E) is covalently linked to a functional group R¹ of the core unit and/or to one or more of the functional groups R² of one or more of the shell units, and
wherein one or more biological targeting molecules (B) may be covalently linked to a functional group R¹ of the core unit and/or to one or more shell units.

12. A polyether polyol dendron conjugate according to any of claims 1 to 10, wherein one effector molecule (E) is covalently linked to the functional group R¹ of the core unit of a second polyether polyol dendron moiety (D), wherein the integer n in the second dendron moiety may also have a value of 0,
wherein one or more molecules of the resulting conjugate are covalently linked to one or more of the functional groups R² of one or more of the shell units of a first polyether polyol dendron moiety according to claim 1,
wherein one biological targeting molecule (B) may be covalently linked to the functional group R¹ of the core unit of the second polyether polyol dendron moiety, and
wherein independently the groups R¹ and R², the core unit and the building block units of the polyether polyol dendron moieties and the integer n may be the same or different for the first polyether polyol dendron moiety and the second polyether polyol dendron moiety.

13. A polyether polyol dendron conjugate according to any of the preceding claims, wherein the polyether polyol dendron moiety (D), the at least one effector molecules (E) and, if present, the biological targeting molecule (B), are covalently linked by linker units (L) independently selected from a direct bond or an aliphatic C₁₋₂₀ hydrocarbon chain, wherein optionally 1-5 non-consecutive methylene groups may be replaced by a group selected from the group comprising -O-, -S-, -C(O) -, C(O)NH-, -NHC(O) -, -NHC(O)NH-, -C(O)O-, -OC(O)O-, -SO₂-, -O-maleinimide-, -O-succinimide-, triazol, aryl, ethene and ethyne, and which may optionally be substituted with one or more groups selected from the group comprising OH, COOH, -O-C₁₋₆-alkyl or phenyl.

14. Kit, comprising one or more polyether polyol dendron conjugates or polyether polyol dendron conjugate precursors according to any of the preceding claims.

15. A polyether polyol dendron conjugate according to any of claims 1 to 13 for therapeutic or diagnostic purposes.

16. A polyether polyol dendron conjugate according to any of claims 1 to 13 for therapeutic or diagnostic purposes in a disease state or condition selected from the group comprising disease states or conditions related to tumors (preferably prostate cancer, breast cancer, lung cancer, cancers of the GI tract), tumor metastases, atherosclerosis, inflammation, (preferably rheumatoid arthritis, osteoarthritis), neoangiogenesis in ophthalmology and benign leasions (preferably benign prostate hyperplasia).

17. Synthesis of a polyether polyol dendron moiety (D) of any of claims 1 to 5, wherein the polyether polyol dendron moiety (D) is built up sequencially from shell to core in a converging synthesis,
wherein two building block units are coupled to the functional groups R² of a core unit or a further building block unit, whereby dendron moiety precursors are obtained, which may again be coupled to the functional groups R² of a core unit or a further building block unit, whereby the process is repeated in order to obtain a dendron moiety with the desired number of generations, thereby doubling the number of exterior R² groups of the growing dendron moiety in each synthesis step.
